(19) Europäisches Patentamt / European Patent Office / Office européen des brevets

(11) **EP 4 600 269 A1**

(12) **EUROPEAN PATENT APPLICATION**
published in accordance with Art. 153(4) EPC

(43) Date of publication:
**13.08.2025 Bulletin 2025/33**

(21) Application number: **23955008.0**

(22) Date of filing: **21.12.2023**

(51) International Patent Classification (IPC):
*C07K 14/78* (2006.01)    *C12N 15/09* (2006.01)
*A61Q 19/00* (2006.01)    *A61K 47/42* (2017.01)
*A61L 15/32* (2006.01)    *A61L 27/24* (2006.01)

(86) International application number:
**PCT/CN2023/140632**

(87) International publication number:
**WO 2025/086447 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.10.2023  CN 202311391711**

(71) Applicant: **Shanxi Jinbo Bio-Pharmaceutical Co., Ltd.**
**Shanxi 030032 (CN)**

(72) Inventors:
• **YANG, Xia**
**Taiyuan, Shanxi 030032 (CN)**
• **LIU, Zengyao**
**Taiyuan, Shanxi 030032 (CN)**
• **LAN, Xiaobin**
**Taiyuan, Shanxi 030032 (CN)**

• **WANG, Lingling**
**Taiyuan, Shanxi 030032 (CN)**
• **ZHANG, Yongjian**
**Taiyuan, Shanxi 030032 (CN)**
• **LIU, Xin**
**Taiyuan, Shanxi 030032 (CN)**
• **ZHANG, Xiaobo**
**Taiyuan, Shanxi 030032 (CN)**
• **HE, Zhenrui**
**Taiyuan, Shanxi 030032 (CN)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

(54) **PREPARATION METHOD FOR BIOSYNTHETIC HUMAN STRUCTURAL MATERIAL TYPE IV COLLAGEN**

(57)    Provided herein is a method of biosynthesizing human body structural material type IV collagen. Provided herein is a recombinant collagen including the sequence represented by SEQ ID NO: 1, etc. The recombinant collagen of the present application exhibits excellent cell - adhesion activity. The method of the present application utilizes genetic engineering techniques to produce the recombinant collagen, thereby overcoming the drawbacks of the prior art.

EP 4 600 269 A1

## Description

The present application claims the priority of the Chinese Invention Patent Application No. 202311391711.1, filed on October 25, 2023, titled "A Method for Preparing Biosynthetic Human Structural Material Type IV Collagen".

## Technical Field

The present disclosure pertains to the field of biomedicine and relates to a recombinant type IV humanized collagen, as well as its preparation method and use.

## Background of the Disclosure

Collagen (abbreviated as COL) is a helical fibrous functional protein constituted by three peptide chains. As the principal component of the extracellular matrix, it is abundant in quantity and widely distributed. In the human body, collagen accounts for 25%-30% of the total protein content and is mainly present in the skin, tendons, and bones. It plays a crucial role in safeguarding and connecting various tissues, exerting significant physiological functions *in vivo.* Type IV collagen is a key protein that, together with laminin, constitutes the basement membrane. Its structural unit is a trimer with a helical structure composed of three $\alpha$ chains. While the subunits of the trimer vary depending on the different tissue, in numerous tissues such as the liver, the trimer consists of two $\alpha$1 chains and one $\alpha$2 chain. The type IV collagen molecule features unique structures at both ends of the TH domain that forms the helical structure. The N-terminal structure is designated as the 7S domain, and the C-terminal structure is termed the NC1 domain.

In recent years, collagen has gained significant attention as a research hotspot due to its favorable biocompatibility, degradability, low antigenicity and unique biological structure. It finds extensive applications in multiple fields such as biomedicine, cosmetics, health products, and food.

There are 28 different types of collagen found in the human body, which can be classified into two major categories: fibrous collagen and non-fibrous collagen, depending on whether their structure is fibrous. Fibrous collagen mainly undertakes the function of a cell scaffold, fixing cell positions and acting as anchors, while also providing tensile strength and stiffness to tissues. Non-fibrous collagen, on the other hand, is further subdivided into basal collagen, short-chain collagen, transmembrane collagen, etc., each fulfilling distinct functions.

The traditional method for producing collagen involves purifying animal-derived tissues through acid-hydrolysis, alkali-hydrolysis, or enzymatic-hydrolysis to extract collagen derivatives. However, the collagen obtained by these methods often loses its original biological activity, exhibits poor water solubility, has difficulty in binding to the human body, confronts challenges in averting the risks of viral infection and sensitization, and is unable to fully perform its true function. Some research institutions have attempted to express human collagen in vitro using conventional recombinant expression methods, but these approaches are costly, time-consuming, and not amenable to large-scale production.

Therefore, there is an urgent demand in the market for a collagen material with excellent biomaterial properties, a high degree of amino - acid sequence homology with the human body, and the capacity for large - scale preparation within an industrial system.

## Summary of the Invention

The inventors carried out a large-scale screening of the functional region of human type IV collagen and identified 11 recombinant collagens. These recombinant collagens can be expressed in *Escherichia coli* and subsequently purified. Furthermore, the inventors found that these recombinant collagens exhibit higher yields, a higher purity of the collagens after fine purification, and higher activity compared to the positive control (bovine type I collagen) in cell adhesion activity assays.

In one aspect, the present disclosure provides a recombinant collagen comprising one or more repeating units linked directly or via a linker, wherein the repeating unit comprises an amino acid sequence selected from the group consisting of SEQ ID NO: 1 (Gakgdkgskgevgfpglagspgipgskgeq) or 28 (Gptgpagqkgepgsdgipgsagekgepglp), or a variant thereof, and wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence.

In one embodiment, the number of repeating units is 2-50 repeating units, such as 2-45, 2-40, 2-35, 2-30, 2-25, 2-20, 2-15, 4-10 or 6-10 repeating units. For example, the number of the repeating units may be 2, 3, 4, 5, 6, 7, 8, 9, 10, 12, 14, 16, 18, 20, 30, 40, or 50, or range therebetween.

In one embodiment, the linker comprises one or more amino acid residues, such as 1-10, 1-9, 1-8, 1-7, 1-6, 1-5, 1-4, 1-3 or 1-2 amino acid residues.

In one embodiment, the mutation is selected from the group consisting of substitution, addition, insertion or deletion.

In one embodiment, the substitution is a conservative amino acid substitution.

In one embodiment, the recombinant collagen is a recombinant human type IV collagen or a recombinant type IV humanized collagen.

In one embodiment, the recombinant collagen has cell adhesion activity.

In one embodiment, the variant of SEQ ID NO: 1 comprises the following mutations: the addition of Gfpgfp (SEQ ID NO: 34) or the N-terminal truncated fragment of SEQ ID NO: 34 with a length of 1-5 amino acid residues to the N-terminus of the amino acid sequence of SEQ ID NO: 1, and/or the addition of GFMGPPGPQGQPGLP (SEQ ID NO: 35) or the C-terminal truncated fragment of SEQ ID NO: 35 with a length of 1-14 amino acid residues to the C-terminus of the amino acid sequence of SEQ ID NO: 1, or the truncation of the amino acid sequence of SEQ ID NO: 1 at C-terminus by 1-5 amino acid residues.

In one embodiment, the variant of SEQ ID NO: 28 comprises the following mutations: the addition of Glpgtp (SEQ ID NO: 36) or the N-terminal truncated fragment thereof with a length of 1-5 amino acid residues to the N-terminus of the amino acid sequence of SEQ ID NO: 28.

In one embodiment, the variant of SEQ ID NO: 1 is selected from the group consisting of SEQ ID NO: 4 (Gakgdkgskgevgfpglagspgipgskgeqgfmgppgpq), 7 (GFPGFPGAKGDKGSKGEVGFPGLAGSPGIPGSK GEQGFMGPPGPQGQPGLP), 10 (Gfpgfpgakgdkgskgevgfpglagspgipgskgeqgfmgppgpq), 13 (Gfpgfpgakgdkgsk-gevgfpglagspgipgskgeqgfm), 16 (Gfpgfpgakgdkgskgevgfpglagspgipgskgeq), 19 (Gakgdkgskgevgfpglagspgipgsk-geqgfmgppgpqgqpglp), 22 (Gakgdkgskgevgfpglagspgipgskgeqgfm), or 31 (Gfpgfpgakgdkgskgevgfpglagspgipgsk).

In one embodiment, the variant of SEQ ID NO: 28 is SEQ ID NO: 25 (Glpgtpgptgpagqkgepgsdgipgsagekgepglp).

In one embodiment, the recombinant collagen comprises an amino acid sequence selected from the group consisting of SEQ ID NOs: 2, 5, 8, 11, 14, 17, 20, 23, 26, 29 and 32, or a variant thereof, wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence.

In one embodiment, the mutation is selected from the group consisting of a substitution, an addition, an insertion or a deletion. In one embodiment, the substitution is a conservative amino acid substitution.

In another aspect, provided is a nucleic acid encoding a recombinant collagen described herein. In one embodiment, the nucleic acid comprises a codon-optimized nucleotide sequence. In one embodiment, the nucleotide sequence is a nucleotide sequence codon-optimized for expression in eukaryotic host cells or prokaryotic host cells, such as yeast or *E. coli*. In one embodiment, the nucleic acid comprises a nucleotide sequence selected from the group consisting of SEQ ID NO: 3, 6, 9, 12, 15, 18, 21, 24, 27, 30 or 33.

In another aspect, provided is a vector comprising a nucleic acid described herein. In one embodiment, the vector comprises an expression control element, nucleotides of a purification tag and/or nucleotides of a leader sequence operably linked to the nucleic acid. In one embodiment, the expression control element is selected from the group consisting of a promoter, a terminator or an enhancer. In one embodiment, the purification tag is selected from the group consisting of a His tag, a GST tag, an MBP tag, a SUMO tag or a NusA tag. In one embodiment, the vector is an expression vector or a cloning vector, preferably pET-28a(+). pET-28a(+) may comprise a HIS, Thrombin, or T7 protein tag at N-terminus, and an His tag at C-terminus. In the present application, the recombinant collagen may comprise an enzyme cleavage site at the N-terminus to facilitate purification.

In another aspect, provided is a host cell comprising a nucleic acid or a vector described herein. In one embodiment, the host cell is a eukaryotic cell or a prokaryotic cell. In one embodiment, the eukaryotic cell is a yeast cell, an animal cell, and/or an insect cell, and in one embodiment, the prokaryotic cell is an *Escherichia coli* cell, such as *E. coli* BL21.

In another aspect, provided is a composition comprising one or more of the recombinant collagens, nucleic acid, vector, and host cell described herein. In one embodiment, the composition is a kit. In one embodiment, the composition is one or more of a biological dressing, a human bionic material, a plastic surgery material or beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive. In one embodiment, the composition is a composition for topical use, injection use, or oral use. In one embodiment, the composition is a composition in the form of a solution, a lyophilized powder, a gel, a sponge, or a fiber.

In another aspect, provided is use of the recombinant collagen, the nucleic acid, the vector, the host cell, and/or the composition of the present application in one or more of a biological dressing, a human bionic material, a plastic surgery or beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive.

In another aspect, provided is a method of promoting cell adhesion, comprising a step of contacting the recombinant collagen, the nucleic acid, the vector, the host cell, and/or the composition of the present application with a cell. In one embodiment, the cell is an animal cell. The animal cell may be a mammalian cell or a human cell. Provided is use of the recombinant collagen, the nucleic acid, the vector, the host cell and/or the composition of the present application in the manufacture of a kit for facilitating cell adhesion.

In another aspect, provided is a beauty method comprising administering the recombinant collagen described herein to a subject, wherein preferably, the administration is a topical administration, oral administration or injection administration, preferably, the subject is a human.

In another aspect, provided is a method of producing the recombinant collagen described herein, comprising:

(1) incubating the host cell described herein under a suitable culture condition;
(2) harvesting the host cell and/or culture medium comprising the recombinant collagen; and
(3) purifying the recombinant collagen.

In one embodiment, the host cell is an *E. coli* cell, preferably an *E. coli* BL21 (DE3) cell.

In one embodiment, step (1) comprises culturing the *E. coli* cell in LB medium and inducing expression with IPTG.

In one embodiment, step (2) comprises harvesting *E. coli* cells, resuspending them in an equilibrium working solution, homogenizing (preferably high-pressure homogenizing) the *E. coli* cells, and isolating the supernatant. In one embodiment, the equilibrium working solution comprises 100-500 mM sodium chloride, 10-50 mM Tris, 10-50 mM imidazole, at pH 7-9.

In one embodiment, step (3) comprises crude purification, enzymatic digestion, fine purification, and/or reverse nickel column purification.

In one embodiment, the crude purification comprises Ni-agarose resin column purification of the supernatant to obtain an eluate containing the target protein, wherein the eluate contains 100-500 mM sodium chloride, 10-50 mM Tris, and 100-500 mM imidazole, preferably at pH 7-9.

In one embodiment, fine purification comprises gradient elution of the eluate containing the target protein with a strong anion exchange chromatography column; wherein preferably, the gradient elution comprises 0-15% solution B for 1-5 minutes followed by holding for 3 column volumes, 15-30% solution B for 1-5 minutes followed by holding for 3 column volumes, 30-50% solution B for 1-5 minutes followed by holding for 3 column volumes, 50-100% solution B for 1-5 minutes followed by holding for 3 column volumes; wherein the solution B contains 10-50 mM Tris, 0.5-5 M sodium chloride, at pH 7-9. In one embodiment, step (3) comprises purifying the recombinant collagen with a purification column, such as a nickel column, and/or cleaving the recombinant collagen with a collagen-processing enzyme.

The advantages of the present disclosure include:

1. The recombinant collagen of the present disclosure is derived from human type IV collagen and is recombinant type IV humanized collagen;
2. The recombinant collagen of the present disclosure is suitable for production by *E. coli* and can be isolated and purified;
3. The recombinant collagen of the present disclosure is produced at high yields and is suitable for subsequent purification (Ni column or strong anion column purification); and
4. The recombinant collagen of the present disclosure has cell adhesion activity. The recombinant collagen of the present disclosure (e.g., C4P7Ch) has higher cell adhesion activity compared to the positive control.

**Brief Description of the Drawings**

Fig. 1 shows the results of electrophoresis detection of C4P7Ca.
Fig. 2 shows the results of electrophoresis detection of C4P7Cb.
Fig. 3 shows the results of electrophoresis detection of C4P7Cc.
Fig. 4 shows the results of electrophoresis detection of C4P7Cd.
Fig. 5 shows the results of electrophoresis detection of C4P7Ce.
Fig. 6 shows the results of electrophoresis detection of C4P7Cf.
Fig. 7 shows the results of electrophoresis detection of C4P7Cg.
Fig. 8 shows the results of electrophoresis detection of C4P7Ch.
Fig. 9 shows the results of electrophoresis detection of C4P7Ea.
Fig. 10 shows the results of electrophoresis detection of C4P7Eb.

Fig. 11 shows the results of electrophoresis detection of C4P7Ec.
Fig. 12 shows the effect of collagen C4P7Ch on cell adhesion.
Fig. 13 shows the effect of collagen C4P7Cf on cell adhesion.

**Detailed Description of the Disclosure**

As used herein, a "recombinant collagen" refers to an amino acid sequence encoded by a designed or modified specific gene, or fragment thereof, or a combination of fragments of such functional amino acid sequence, which is prepared by recombinant DNA technology. The gene encoding sequence or amino acid sequence of a recombinant collagen may have a low homology with the gene encoding sequence or amino acid sequence of human collagen. A "recombinant humanized collagen" refers to the full-length or partial amino acid sequence fragment encoded by a specific type gene of human collagen prepared by recombinant DNA technology, or a combination containing functional fragments of human collagen. Herein, the recombinant collagen comprises one or more repeating units. The repeating unit may be derived from human type IV collagen. Thus, the recombinant collagen may be recombinant type IV humanized collagen. The plurality of repeating units may be linked via a linker, which may be a natural amino acid residue(s), for example 1-50 amino acid residues, of the repeating units on human type IV collagen. A repeating unit may be SEQ ID NO: 1, 4, 7, 10, 13, 16, 19, 22, 25, 28, 31, or 33. A recombinant collagen may be SEQ ID NO: 2, 5, 8, 11, 14, 17, 20, 23, 26, 29, or 32.

As used herein, the term "variant" means a recombinant collagen having cell adhesion activity that includes alterations/mutations (i.e., substitutions, additions, insertions, and/or deletions) at one or more positions. Substitution means the replacement of an amino acid occupying a certain position with a different amino acid; deletion means the removal of an amino acid occupying a certain position; and insertion means the addition of an amino acid adjacent to and immediately after an amino acid occupying a certain position. Addition refers to the addition of one or more amino acid residues to the C-terminus and/or N-terminus of an amino acid sequence. The substitution may be a conservative substitution. A variant of a repeating unit may be a sequence in which one or more amino acid residues in SEQ ID NO: 1, 4, 7, 10, 13, 16, 19, 22, 25, 28, 31 or 33 are changed or mutated (i.e., substituted, added, inserted and/or deleted). A variant of a recombinant collagen may be a sequence in which one or more amino acid residues in SEQ ID NO: 2, 5, 8, 11, 14, 17, 20, 23, 26, 29 or 32 are changed or mutated (i.e., substituted, added, inserted and/or deleted).

For example, a variant of a repeating unit of SEQ ID NO: 1 may be a variant comprising the following mutations: the addition of Gfpgfp (SEQ ID NO: 34) or the N-terminal truncated fragment of SEQ ID NO: 34 (for example 1-5 amino acid residues, such as 1, 2, 3, 4 or 5 amino acid residues are truncated from the N-terminus of SEQ ID NO: 34, corresponding to fpgfp, pgfp, gfp, fp and p residues, respectively) to the N-terminus of the amino acid sequence of SEQ ID NO: 1, and/or the addition of GFMGPPGPQGQPGLP (SEQ ID NO: 35) or a C-terminal truncated fragment of SEQ ID NO: 35 (1-14 amino acid residues, e.g., 1, 2, 3, 4, 5, 6, 7, 8, 9, 10, 11, 12, 13 or 14 amino acid residues, are truncated from the C-terminus of SEQ ID NO: 35) to the C-terminus of the amino acid sequence of SEQ ID NO: 1. A variant of a repeating unit of SEQ ID NO: 1 may be a variant comprising the following mutations: the addition of Gfpgfp (SEQ ID NO: 34) or the N-terminal truncated fragment thereof (for example 1, 2, 3, 4 or 5 amino acid residues are truncated from the N-terminus of SEQ ID NO: 34) to the N-terminus of the amino acid sequence of SEQ ID NO: 1, and/or truncation of 1-5 amino acid residues, such as 1, 2 or 3 amino acid residues, at the C-terminus of the amino acid sequence of SEQ ID NO: 1.

A variant of a repeating unit of SEQ ID NO: 28 may be a variant comprising the following mutations: the addition of Glpgtp (SEQ ID NO: 36) or the N-terminal truncated fragment thereof (for example 1-5 amino acid residues, e.g., 1, 2, 3, 4 or 5 amino acid residues are truncated from the N-terminus of SEQ ID NO: 36) to the N-terminus of the amino acid sequence of SEQ ID NO: 28.

In the context of the present disclosure, a conservative substitution may be defined by substitution within one or more of the amino acid classes reflected in one or more of the following tables:

Conservative classes of amino acid residues:

| | |
|---|---|
| Acidic residues | D and E |
| Basic residues | K, R and H |
| Hydrophilic uncharged residues | S, T, N, and Q |
| Aliphatic uncharged residues | G, A, V, L and I |
| Non-polar uncharged residues | C, M and P |
| Aromatic residues | F, Y, and W. |

Alternative physical and functional classification of amino acid residues:

| | |
|---|---|
| Alcohol group-containing residues | S and T |
| Aliphatic Residues | I, L, V and M |
| Cycloalkenyl-related residues | F, H, W and Y |
| Hydrophobic residues | A, C, F, G, H, I, L, M, R, T, V, W and Y |
| Negatively charged residues | D and E |
| Polar residues | C, D, E, H, K, N, Q, R, S and T |
| Positively charged residues | H, K and R |
| Small residues | A, C, D, G, N, P, S, T and V |
| Minimal residues | A, G and S |
| Residues involved in turn formation | A, C, D, E, G, H, K, N, Q, R, S, P and T |
| Flexible residues | Q, T, K, S, G, P, D, E, and R. |

As used herein, "cell adhesion" refers to the adhesion between cells and collagen. Collagen, such as the recombinant collagen described herein, can facilitate adhesion between cells and the container in which the cells are cultured.

As used herein, the term "expression" includes any step involved in the production of recombinant collagen, including, but not limited to, transcription, post-transcriptional modification, translation, post-translational modification, and secretion.

As used herein, the term "expression vector" means a linear or circular DNA molecule comprising a polynucleotide which encodes a recombinant collagen protein and is operably linked to a control sequence provided for its expression.

As used herein, the term "host cell" means any cell type amenable to transformation, transfection, transduction, etc. with a nucleic acid construct or expression vector comprising a polynucleotide of the present disclosure. The term "host cell" encompasses any progeny of a parent cell that is not identical to the parent cell due to a mutation occurring during replication.

As used herein, the term "nucleic acid" means a single-stranded or double-stranded nucleic acid molecule that is isolated from a naturally occurring gene, or is modified in a manner which is otherwise not present in nature to contain a segment of a nucleic acid, or is synthetic, and the nucleic acid molecule may comprise one or more control sequences. The nucleic acid may be SEQ ID NO: 3, 6, 9, 12, 15, 18, 21, 24, 27, 30 or 33. The nucleic acid may be a codon-optimized nucleic acid, for example, a nucleic acid that is codon-optimized for expression in *E. coli* cells.

The term "operably linked" means a configuration in which a control sequence is placed at an appropriate position relative to an encoding sequence of a polynucleotide such that the control sequence directs the expression of the encoding sequence.

The degree of association between two amino acid sequences or between two nucleotide sequences is described by the parameter "sequence identity". For the purposes of this disclosure, the sequence identity between two amino acids is determined by Needleman-Wunsch Algorithm implemented by the Needle program (Needleman and Wunsch, 1970, J. Mol. Biol. 48: 443-453) of the EMBOSS software package (EMBOSS: The European Molecular Biology Open Software Suite, Rice et al, 2000, Trends Genet. 16: 276-277) (Version 5.0.0 or later is preferred). The parameters used are the gap opening penalty of 10, the gap extension penalty of 0.5, and the EBLOSUM62 (EMBOSS version of BLOSUM62) substitution matrix. Needle's output labeled "Longest Identity" (obtained using the non-simplified option) is used as the percent identity and calculated as follows:

$$\text{(identical residues x 100) / (alignment length - total number of gaps in the alignment)}$$

For the purposes of this disclosure, the sequence identity between two deoxynucleotide sequences is determined by the Needleman-Wunsch Algorithm implemented by the Needle program (Needleman and Wunsch, 1970) of the EMBOSS software package (EMBOSS: The European Molecular Biology Open Software Suite, Rice et al, 2000) (Version 5.0.0 or later is preferred). The parameters used are the gap opening penalty of 10, the gap extension penalty of 0.5, and the EDNAFULL (EMBOSS version of NCBI NUC4.4) substitution matrix. Needle's output labeled "Longest Identity" (obtained using the non-simplified option) is used as the percent identity and calculated as follows:

(identical deoxyribonucleotides x 100) / (alignment length - total number of gaps in the alignment)

**Recombinant collagen**

The present disclosure provides a recombinant collagen comprising one or more repeating units linked directly or via a linker, wherein the repeating units comprise an amino acid sequence selected from SEQ ID NO: 1, 4, 7, 10, 13, 16, 19, 22, 25, 28, 31 or 33, or a variant thereof. The variant may be (1) an amino acid sequence in which one or more amino acid residues are mutated in the amino acid sequence of SEQ ID NO: 1, 4, 7, 10, 13, 16, 19, 22, 25, 28, 31 or 33, or (2) an amino acid sequence having at least 80%, 85%, 86%, 87%, 88%, 89%, 90%, 91%, 92%, 93%, 94%, 95%, 96%, 97%, 98%, 99%, or 100% identity with the amino acid sequence of SEQ ID NO: 1, 4, 7, 10, 13, 16, 19, 22, 25, 28, 31, or 33. With respect to the recombinant collagen described herein, the mutation may be selected from the group consisting of a substitution, addition, insertion, or deletion. Preferably, the substitution is a conservative amino acid substitution.

The recombinant collagen described herein may comprise a plurality of repeating units, for example, 2-50 repeating units, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 repeating units.

The linker in the recombinant collagen described herein may comprise one or more amino acid residues, for example, 1-10, 1-9, 1-8, 1-7, 1-6, 1-5, 1-4, 1-3, or 1-2 amino acid residues.

The recombinant collagen is recombinant human type IV collagen or recombinant type IV humanized collagen, preferably having cell adhesion activity. The recombinant collagen described herein may be human-derived, and thus is a recombinant type IV humanized collagen.

The recombinant collagen described herein may also comprise an amino acid sequence selected from the group consisting of SEQ ID NO: 2, 5, 8, 11, 14, 17, 20, 23, 26, 29 or 32, or a variant thereof, wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence.

**Nucleic acid constructs**

The present disclosure also relates to a nucleic acid construct comprising a nucleic acid of the present disclosure operably linked to one or more control sequences that direct the expression of an encoding sequence in a suitable host cell under a condition compatible with the control sequences. The vector may comprise a nucleic acid construct.

Nucleic acids can be modified in a variety of ways to enable the expression of recombinant collagen. Depending on the expression vector, it may be desirable or necessary to modify the nucleic acid prior to its insertion into the vector. Techniques for modifying nucleic acids using recombinant DNA methods are well known in the art.

The control sequence may be a promoter, i.e., it is recognized by a host cell for the expression of a polynucleotide encoding a recombinant collagen of the present disclosure. The promoter comprises transcriptional control sequences that mediate the expression of recombinant collagen. The promoter can be any nucleic acid that exhibits transcriptional activity in a host cell, including variants, truncated or hybrid promoters, and can be obtained from a gene encoding an extracellular or intracellular recombinant collagen homologous or heterologous to the host cell.

Examples of suitable promoters for directing transcription of the vectors or nucleic acid constructs of the disclosure in bacterial host cells are promoters obtained from: *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltoamylase gene (*amyM*), *Bacillus subtilis* fructan sucrase gene (*sacB*), *Bacillus subtilis xylA* and *xylB* genes, *Bacillus thuringiensis cryIIIA* gene, *E. coli* lac operon, and *E. coli* trc promoter.

In yeast hosts, useful promoters are obtained from the following genes: *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triosephosphate isomerase (TPI), *Saccharomyces cerevisiae* metallothionein (CUP1), and *Saccharomyces cerevisiae* 3-phosphoglycerate kinase.

The control sequence may also be a transcription terminator recognized by the host cell to terminate transcription. The terminator may be operably linked to the 3' end of the polynucleotide encoding the recombinant collagen. Any terminator that is functional in the host cell may be used in the present disclosure.

Preferred terminators for bacterial host cells are obtained from the genes of *Bacillus clausii* alkaline protease (*aprH*), *Bacillus licheniformis* alpha-amylase (*amyL*), and *E. coli* ribosomal RNA (*rrnB*).

Preferred terminators for yeast host cells are obtained from the following genes: *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al. (1992).

The control sequence may also be an mRNA stabilizer region downstream of the promoter and upstream of the encoding sequence of the gene, which increases the expression of the gene.

Examples of suitable mRNA stabilizer regions are obtained from the following genes: *Bacillus thuringiensis cryIIIA* gene (WO 94/25612) and *Bacillus subtilis* SP82 gene (Hue et al., 1995, Journal of Bacteriology 177: 3465-3471).

The control sequence may also be a leader sequence, i.e., a non-translated region of an mRNA that is important for translation in the host cell. The leader sequence is operably linked to the 5' end of the polynucleotide encoding the recombinant collagen. Any leader sequence that is functional in the host cell may be used.

Suitable leader sequences for yeast host cells are obtained from the following genes: *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, which is operably linked to the 3' end of the polynucleotide and when transcribed, is recognized by the host cell as a signal to add polyadenylate residues to the transcribed mRNA. Any polyadenylation sequence that is functional in the host cell may be used.

Useful polyadenylation sequences for yeast host cells are described by Guo and Sherman, 1995, Mol. Cellular Biol. 15: 5983-5990.

The control sequence may also be a signal peptide encoding region that encodes a signal peptide linked to the N-terminus of the recombinant collagen and directs the recombinant collagen into the secretory pathway of the cell. The 5'-end of the encoding sequence of the polynucleotide may itself contain a signal peptide encoding sequence that is naturally linked in open reading frame to the segment of the encoding sequence encoding the recombinant collagen. Alternatively, the 5'-end of the encoding sequence may contain a signal peptide encoding sequence that is foreign to the encoding sequence. In cases where the encoding sequence does not naturally contain a signal peptide encoding sequence, a foreign signal peptide encoding sequence may be required. Alternatively, the foreign signal peptide encoding sequence may simply replace the natural signal peptide encoding sequence in order to enhance the secretion of the recombinant collagen. However, any signal peptide encoding sequence that directs the expressed recombinant collagen into the secretory pathway of the host cell may be used.

An effective signal peptide encoding sequence for a bacterial host cell is a signal peptide encoding sequence obtained from the following genes: *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral protease (*nprT, nprS, nprM*) and *Bacillus subtilis prsA*. Additional signal peptides are described by Simonen and Palva, 1993, Microbiological Reviews 57: 109-137.

Useful signal peptides for yeast host cells are obtained from the following genes: *Saccharomyces cerevisiae* alpha-factor and *Saccharomyces cerevisiae* invertase. Other useful signal peptide encoding sequences are described by Romanos et al. (Yeast 8: 423-488).

## Expression vector

The disclosure also relates to recombinant expression vectors comprising a nucleic acid, a promoter, and transcription and translation termination signals of the present disclosure. Nucleic acids and control sequences may be ligated together to produce recombinant expression vectors that may include one or more convenient restriction sites for insertion or substitution of a polynucleotide encoding the recombinant collagen at such sites. Alternatively, the polynucleotide may be expressed by inserting a nucleic acid, or a nucleic acid construct comprising the nucleic acid into an appropriate vector for expression. When an expression vector is produced, the encoding sequence is located in the vector such that the encoding sequence is operably linked to an appropriate control sequence for expression.

A recombinant expression vector may be any vector (e.g., a plasmid or virus) that can be conveniently subjected to a recombinant DNA procedure and may enable the expression of a polynucleotide. The choice of the vector will typically depend on the compatibility of the vector with the host cell into which the vector is to be introduced. The vector may be a linear chain or a closed circular plasmid.

The vector may be an autonomously replicating vector, i.e. a vector that exists as an extrachromosomal entity, whose replication is independent of chromosomal replication, such as a plasmid, an extrachromosomal element, a minichromosome or an artificial chromosome. The vector may contain any means for ensuring self-replication. Alternatively, the vector may be one that, when introduced into a host cell, integrates into the genome and replicates with one or more chromosomes into which it has been integrated. Furthermore, separate vectors or plasmids or two or more vectors or plasmids, which collectively contain the total DNA to be introduced into the genome of the host cell may be used, or transposons may be used.

The vector preferably contains one or more selectable markers that allow for convenient selection of transformed cells, transfected cells, transduced cells and the like. A selectable marker is a gene whose product provides a biocide resistance or a virus resistance, a resistance to heavy metals, a prototrophy to auxotroph, etc.

Examples of bacterial selectable markers are the *dal* gene from *Bacillus licheniformis* or *Bacillus subtilis,* or markers conferring antibiotic resistance, such as ampicillin, chloramphenicol, kanamycin, neomycin, spectinomycin, or tetracycline resistance. Suitable markers for yeast host cells include, but are not limited to: ADE2, HIS3, LEU2, LYS2, MET3, TRP1, and URA3.

The selectable marker may be a dual selectable marker system as described in WO 2010/039889. In one aspect, the dual selectable marker is an *hph-tk* dual selectable marker system.

The vector may contain an element that allows integration of the vector into the genome of the host cell or autonomous replication of the vector in the cell independent of the genome.

For integration into the host cell genome, the vector may rely on the polynucleotide sequence encoding the recombinant collagen or any other element of the vector for integration into the genome by homologous or non-homologous recombination. Alternatively, the vector may contain an additional polynucleotide for directing integration by homologous recombination into the genome of the host cell at a precise location(s) in the chromosome(s). To increase the likelihood of integration at a precise location, the integrational element should contain a sufficient number of nucleic acids, such as 100 to 10,000 base pairs, 400 to 10,000 base pairs, and 800 to 10,000 base pairs, which have a high degree of sequence identity to the corresponding target sequences to enhance the probability of homologous recombination. The integrational element may be any sequence homologous to a target sequence within the genome of the host cell. Furthermore, the integrational element may be a non-encoding or encoding polynucleotide. In another aspect, the vector can be integrated into the genome of the host cell by non-homologous recombination.

In order to replicate autonomously, the vector may further comprise an origin of replication enabling the vector to replicate autonomously in the host cell in question. The origin of replication may be any plasmid replicator that functions in cells to mediate autonomous replication. The term "origin of replication" or "plasmid replicator" means a polynucleotide that enables a plasmid or vector to replicate in vivo.

Examples of bacterial origins of replication are the origins of replication of plasmids pBR322, pUC19, pACYC177, and pACYC184 that allow replication in *E. coli,* as well as the origins of replication of plasmids pUB110, pE194, pTA1060, and pAMβ1 that allow replication in *Bacillus.*

Examples of origins of replication for use in yeast host cells are 2-micron origin of replication, ARS1, ARS4, the combination of ARS1 and CEN3, as well as the combination of ARS4 and CEN6.

More than one copy of the polynucleotide of the present disclosure can be inserted into a host cell to increase the production of recombinant collagen. An increased number of copies of a polynucleotide can be obtained by integrating at least one additional copy of the sequence into the host cell genome or by including an amplifiable selectable marker gene together with the polynucleotide where cells comprising amplified copies of the selectable marker gene and thus additional copies of the polynucleotide can be selected by culturing the cells in the presence of an appropriate selectable agent.

Procedures for ligating the elements described above to construct the recombinant expression vectors of the present disclosure are well known to those of ordinary skill in the art (see, for example, Sambrook et al., 1989, Molecular Cloning: A Laboratory Manual (2nd ed., Cold Spring Harbor Laboratory Press, Cold Spring Harbor, NY)).

## Host cell

The present disclosure also relates to recombinant host cells comprising a polynucleotide of the present disclosure operably linked to one or more control sequences that direct the production of the recombinant collagen of the present disclosure. The construct or vector comprising the polynucleotide is introduced into the host cell such that the construct or vector is maintained as a chromosomal integrant or as an autonomously replicating extrachromosomal vector, as described earlier. The term "host cell" encompasses any progeny of a parent cell that is not identical to the parent cell due to a mutation occurring during replication. The choice of host cell will depend largely on the gene encoding the recombinant collagen and its source.

The host cell may be any cell useful in the recombinant production of the recombinant collagen of the present disclosure, for example, a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, Escherichia coli, Flavobacterium, Fusobacterium, Helicobacter, Ilyobacter, Neisseria, Pseudomonas, Salmonella,* and *Ureaplasma.*

The host cell may also be a cell of a eukaryotic organism, such as a mammalian, insect, plant or fungus. Plant cells herein do not include plant cells that can regenerate into a whole plant. Animal cells also do not include cells that can generate an animal body.

The host cells may be fungal cells such as *Basidiomycota, Chytridiomycota, Zygomycota,* and *Oomycota,* among others. The host cell may be a yeast cell, including *ascosporogenous* yeast (*Endomycetales*), *basidiosporogenous* yeast, and yeast belonging to *Fungi Imperfecti* (*Blastomycetes*). The yeast host cell may be a cell of *Candida, Hansenula, Kluyveromyces, Pichia, Saccharomyces, Schizosaccharomyces* or *Yarrowia,* such as a cell of *Kluyveromyces lactis, Saccharomyces carlsbergensis, Saccharomyces cerevisiae, Saccharomyces diastaticus, Saccharomyces douglasii,*

*Saccharomyces kluyveri, Saccharomyces norbensis, Saccharomyces oviformis* or *Yarrowia lipolytica.*

**Production Method**

The present disclosure also relates to a method of producing the recombinant collagen as described herein, comprising:

(1) incubating the host cell described herein under a suitable culture condition;
(2) harvesting the host cell and/or culture medium comprising the recombinant collagen; and
(3) purifying the recombinant collagen.

Using methods known in the art, the host cells are cultured in a suitable nutrient medium for the production of recombinant collagen. For example, cells can be cultured in shake flask, or by small- or large-scale fermentation (including continuous, batch, fed-batch or solid-state fermentation) in a laboratory or industrial fermenter, wherein the cultivation is performed in a suitable medium and under conditions that allow expression and/or isolation of the recombinant collagen. The cultivation is performed in a suitable nutrient medium comprising carbon and nitrogen sources and inorganic salts using procedures known in the art. Suitable media may be obtained from commercial suppliers or may be prepared according to disclosed compositions (e.g., in the catalogs of American Type Culture Collection). If a recombinant collagen is secreted into the nutrient medium, the recombinant collagen can be recovered directly from the medium. If a recombinant collagen is not secreted, it can be recovered from the cell lysate.

Recombinant collagen can be detected using methods known in the art that are specific for recombinant collagen. These detection methods include, but are not limited to, the use of specific antibodies. For example, an adhesion assay can be used to determine the activity of the recombinant collagen.

The recombinant collagen can be recovered by methods known in the art. For example, the recombinant collagen can be recovered from the nutrient medium by conventional procedures including, but not limited to, collection, centrifugation, filtration, extraction, spray drying, evaporation, or precipitation. In one aspect, the recombinant collagen is recovered from the fermentation broth comprising recombinant collagen.

Recombinant collagen can be purified by known procedures in the art, including, but not limited to, chromatography (e.g., ion exchange chromatography, affinity chromatography, hydrophobic chromatography, focusing chromatography, and size exclusion chromatography), electrophoretic procedures (e.g., preparative isoelectric focusing electrophoresis), differential solubility (e.g., ammonium sulfate precipitation), SDS-PAGE, or extraction to obtain substantially pure recombinant collagen.

Step (1) may comprise one or more of the following steps: constructing an expression plasmid, e.g., inserting an encoding nucleotide sequence into a pET-28a-Trx-His expression vector to obtain a recombinant expression plasmid. Successfully constructed expression plasmids can be transformed into *E. coli* cells (e.g., *E. coli* competent cells BL21 (DE3)). The specific process can be as follows: (1) taking the plasmid to be transformed and adding it to *E. coli* competent cells BL21 (DE3); (2) placing the mixture on ice in an ice bath (e.g., for 10-60 min, e.g., 30 min), followed by a heat shock in a water bath (e.g., at 40-50°C, e.g., 42°C, for 45-90 s), taking out the mixture and placing it on ice in an ice bath (e.g., for 1-5 min, e.g., 2 min); (3) adding liquid LB medium followed by an incubation (e.g., at 35-40°C, e.g., 37°C, at 150-300 rpm, e.g., 220 rpm for 40-80 min, e.g., 60 min); (4) spreading the bacterial solution and picking single colonies. For example, the bacterial solution is taken and evenly spread on LB plates containing ampicillin sodium, and the plates are cultured in an incubator at 37°C for 15-17 hours until uniform-sized colonies grow in the plates.

Step (2) may comprise culturing the single colonies in LB medium containing an antibiotic stock (e.g., in a shaker at 150-300 rpm, e.g., 220 rpm, at a constant temperature of 35-40°C, e.g., 37°C for 5-10 hours, e.g., 7 hours). Then, the cultured shake flask is cooled to 10-20°C, for example 16°C, and IPTG is added to induce expression for a period of time, and then the cells are collected (for example, by centrifugation).

Step (3) may include resuspending the bacterial cells with an equilibrium working solution, cooling the bacterial liquid to ≤ 15°C, performing homogenization (e.g., high-pressure homogenization, e.g., 1-5 times, e.g., 2 times), and separating the homogenized bacterial liquid to obtain a supernatant. The equilibrium working solution may comprise 100-500 mM sodium chloride, 10-50 mM Tris, and 10-50 mM imidazole, at pH 7-9. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 10, 15, 20, 25, 30, 35, 40, 45, or 50 nM. The pH may be 7, 7.5, 8, 8.5, or 9.

Step (3) may include purifying and enzymatically digesting the recombinant collagen. The purification may be a crude purification comprising a Ni-agarose column purification of the supernatant to obtain an eluate containing the target protein. Crude purification may comprise washing the column material with water, for example for 2-10 column volumes (CVs), for example 5 CVs. The column material can be equilibrated with an equilibration solution (200 mM sodium chloride, 25 mM Tris, 20 mM imidazole at pH 8.0), for example for 2-10 CVs, for example 5 CVs. The equilibrium solution may comprise 100-500 mM sodium chloride, 10-50 mM Tris, and 10-50 mM imidazole at pH 7-9. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 10, 15, 20, 25, 30, 35, 40, 45, or 50 nM. The pH may be 7, 7.5, 8, 8.5, or 9.

Step (3) may comprise loading the supernatant onto the column material and washing the impurity proteins with a washing solution. The washing solution may comprise 100-500 mM sodium chloride, 10-50 mM Tris, and 10-50 mM imidazole at pH 7-9. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 10, 15, 20, 25, 30, 35, 40, 45, or 50 nM. The pH may be 7, 7.5, 8, 8.5, or 9. Then, the eluent can be added and the flow-through liquid is collected. The eluent may comprise 100-500 mM sodium chloride, 10-50 mM Tris, 100-500 mM imidazole, at pH 8.0. For example, the concentration of sodium chloride may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The concentration of Tris may be 10, 15, 20, 25, 30, 35, 40, 45, or 50 nM. The concentration of imidazole may be 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, or 490 nM. The pH may be 7, 7.5, 8, 8.5, or 9.

The enzymatic digestion may comprise adding a collagen-processing enzyme for enzymatic digestion (at a ratio of the total amount of protein to the total amount of collagen-processing enzyme of 10-100: 1, e.g., 50: 1, at 10-20°C, e.g., 16°C for 2-8 h, e.g., 4 h). The enzymatically digested protein solution is dialyzed, for example, in a dialysis bag at 1-6°C, for example 4°C for 1-8 hours, for example 2 hours, and then transferred to a new dialysate for dialysis at 1-6°C, for example 4°C overnight.

Purification may include fine purification (e.g., for proteins with an isoelectric point > 8.0). Preferably, the fine purification comprises using a strong anion exchange chromatography column (e.g., the pH may be 7, 7.5, 8, 8.5 or 9.) The eluate containing the target protein or the product after enzymatic digestion (e.g., enzymatically digested and dialyzed product) is subjected to gradient elution. The gradient elution comprises 0-15% solution B for 1-5 minutes followed by holding for 1-5 column volumes, e.g., 3 column volumes, 15-30% solution B for 1-5 minutes followed by holding for 1-5 column volumes, e.g., 3 column volumes, 30-50% solution B for 1-5 minutes followed by holding for 1-5 column volumes, e.g., 3 column volumes, 50-100% solution B for 1-5 minutes followed by holding for 1-5 column volumes, e.g., 3 column volumes. Solution B may comprise 10-50 mM Tris, 0.5-5 M sodium chloride, at pH 7-9. For example, the concentration of Tris is 15, 20, 25, 30, 35, 40, or 45 mM. The concentration of sodium chloride is 1, 2, 3, or 4 M. The pH may be 7, 7.5, 8, 8.5, or 9. Fine purification may include equilibrating the column material with solution A and loading, followed by gradient elution. Solution A may contain 10-50 mM Tris, 10-50 mM sodium chloride, at pH 7-9. For example, the concentration of Tris is 15, 20, 25, 30, 35, 40, or 45 mM. The concentration of sodium chloride is 15, 20, 25, 30, 35, 40 or 45 mM. The pH may be 7, 7.5, 8, 8.5, or 9.

To clarify the purpose, technical solutions, and advantages of the present disclosure, the technical solutions in the examples will be clearly and completely described below in conjunction with the following examples. Obviously, the described examples are partial embodiments of the present disclosure, rather than all of the embodiments. Based on the examples of the present disclosure, all other examples obtained by ordinary technicians in the art without making any creative work fall within the scope of protection of the present disclosure.

The following examples are further provided to illustrate the present disclosure.

## Examples

The present disclosure is further illustrated by the following examples, but any example or combination thereof should not be construed as limiting the scope of the present disclosure. The scope of the present disclosure is defined by the following claims. As a person skilled in the art combines this specification with common knowledge in the art, they can clearly discern the scope defined by the claims. Without departing from the spirit and scope of the present disclosure, those skilled in the art can make modifications to the technical solution of the present disclosure. Such modifications also fall within the scope of the present disclosure.

## Example 1: Construction, Expression and Screening of Recombinant Type IV Humanized Collagen Fragments

1. Large-scale screening for functional regions was carried out to obtain the following different target gene functional regions of recombinant type IV humanized collagen.

1) The amino acid sequence of C4P7Ch (the amino acid sequence of repeating unit is Gakgdkgskgevgfpglagspgipgskgeq, SEQ ID NO: 1, and the number of repeating units is 10; the amino acid sequence of C4P7Ch is SEQ ID NO: 2):

|Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

Gakgdkgskgevgfpglagspgipgskgeq

C4P7Ch base sequence (SEQ ID NO: 3):

GGGGCTAAAGGAGACAAGGGCAGCAAGGGCGAAGTCGGTTTCCCAGGTCTGGCTGGTAGCCCG

GGCATCCCGGGTTCAAAGGGTGAACAAGGTGCTAAAGGCGACAAAGGCAGCAAGGGTGAGGT

TGGTTTCCCGGGTCTGGCGGGTTCTCCAGGCATCCCGGGTAGCAAAGGAGAACAAGGTGCGAA

AGGCGATAAAGGCTCCAAGGGTGAAGTGGGCTTCCCGGGTTTAGCCGGTAGCCCAGGTATTCC

GGGTAGCAAAGGCGAACAGGGTGCGAAAGGCGACAAAGGGAGTAAGGGCGAGGTGGGTTTTC

CGGGTTTGGCTGGCTCGCCGGGTATTCCGGGTTCAAAGGGCGAACAGGGCGCGAAAGGTGATA

AAGGCAGCAAAGGCGAGGTTGGCTTCCCGGGTCTGGCAGGTAGCCCGGGTATCCCGGGTAGCA

AGGGTGAGCAGGGTGCCAAAGGCGACAAAGGTAGCAAGGGGGAAGTGGGTTTTCCGGGACTG

GCAGGTAGCCCGGGTATCCCGGGTTCTAAGGGCGAGCAGGGTGCGAAAGGTGACAAAGGTAG

CAAGGGCGAGGTTGGCTTTCCGGGCTTGGCGGGTAGCCCGGGCATTCCGGGCTCCAAGGGTGA

ACAAGGTGCGAAAGGTGATAAAGGCTCTAAGGGTGAGGTTGGTTTTCCGGGTCTGGCGGGTTC

CCCGGGCATTCCGGGCTCGAAGGGCGAGCAAGGTGCTAAAGGTGATAAGGGCTCCAAGGGCG

AGGTGGGTTTCCCGGGCCTGGCAGGCTCTCCGGGCATCCCGGGTTCGAAGGGCGAACAGGGTG

CGAAAGGCGATAAAGGTTCCAAGGGCGAAGTCGGATTCCCTGGCCTCGCCGGTAGCCCGGGCA

TCCCTGGCTCCAAGGGCGAGCAG

2) C4P7Cf (the amino acid sequence of repeating unit is Gakgdkgskgevgfpglagspgipgskgeqgfmgppgpq, SEQ ID NO: 4, and the number of repeating units is 8; the amino acid sequence of C4P7Cf is SEQ ID NO: 5):

|Gakgdkgskgevgfpglagspgipgskgeqgfmgppgpq

Gakgdkgskgevgfpglagspgipgskgeqgfmgppgpq

Gakgdkgskgevgfpglagspgipgskgeqgfmgppgpq

Gakgdkgskgevgfpglagspgipgskgeqgfmgppgpq

Gakgdkgskgevgfpglagspgipgskgeqgfmgppgpq

Gakgdkgskgevgfpglagspgipgskgeqgfmgppgpq

Gakgdkgskgevgfpglagspgipgskgeqgfmgppgpq

Gakgdkgskgevgfpglagspgipgskgeqgfmgppgpq

C4P7Cf base sequence (SEQ ID NO: 6):

GGAGCTAAAGGGGACAAAGGCTCCAAAGGCGAAGTCGGTTTCCCGGGCCTGGCGGGTAG
CCCGGGTATCCCGGGTAGCAAGGGTGAGCAGGGGTTCATGGGTCCACCGGGCCCACAGGGTGC
CAAAGGTGATAAAGGTTCTAAGGGCGAGGTGGGTTTCCCGGGGCTGGCGGGTTCTCCGGGCAT
TCCGGGAAGCAAGGGTGAACAGGGCTTTATGGGTCCGCCAGGTCCGCAGGGTGCGAAAGGTGA
TAAAGGCAGCAAGGGAGAAGTTGGCTTCCCGGGCCTGGCAGGCAGCCCGGGCATTCCGGGGTC
GAAGGGCGAACAAGGTTTCATGGGTCCGCCTGGTCCGCAAGGTGCGAAAGGTGATAAGGGTAG
CAAGGGTGAAGTGGGTTTTCCGGGATTAGCGGGTTCTCCGGGCATTCCGGGTTCAAAAGGTGA
ACAAGGCTTTATGGGTCCGCCTGGCCCGCAGGGTGCTAAAGGCGACAAGGGTAGCAAAGGCGA
GGTAGGTTTCCCGGGTTTGGCGGGCAGCCCGGGCATTCCGGGTTCCAAGGGCGAGCAGGGTTTT
ATGGGCCCACCGGGCCCGCAAGGCGCAAAAGGTGATAAGGGCAGCAAAGGCGAGGTGGGCTT

CCCGGGACTGGCAGGTTCTCCGGGTATCCCGGGTTCCAAGGGTGAGCAGGGTTTCATGGGCCC
ACCGGGTCCGCAGGGTGCGAAAGGCGACAAAGGTAGCAAGGGCGAAGTTGGTTTTCCGGGCCT
GGCTGGTTCGCCGGGCATCCCGGGCTCCAAGGGCGAGCAAGGCTTCATGGGTCCACCGGGTCC
GCAAGGTGCCAAAGGCGACAAAGGTAGCAAGGGCGAGGTTGGTTTTCCGGGCTTGGCTGGTAG
CCCTGGCATCCCGGGGTCCAAGGGTGAACAGGGCTTTATGGGTCCGCCGGGCCCTCAA

3) C4P7Ca amino acid sequence (the amino acid sequence of the repeating unit is GFPGFPGAKGDKGSKGEV GFPGLAGSPGIPGSKGEQGFMGPPGPQGQPGLP, SEQ ID NO: 7, and the number of repeating units is 6; the amino acid sequence of C4P7Ca is SEQ ID NO: 8):

GFPGFPGAKGDKGSKGEVGFPGLAGSPGIPGSKGEQGFMGPPGPQGQPGLP

GFPGFPGAKGDKGSKGEVGFPGLAGSPGIPGSKGEQGFMGPPGPQGQPGLP

GFPGFPGAKGDKGSKGEVGFPGLAGSPGIPGSKGEQGFMGPPGPQGQPGLP

GFPGFPGAKGDKGSKGEVGFPGLAGSPGIPGSKGEQGFMGPPGPQGQPGLP

GFPGFPGAKGDKGSKGEVGFPGLAGSPGIPGSKGEQGFMGPPGPQGQPGLP

GFPGFPGAKGDKGSKGEVGFPGLAGSPGIPGSKGEQGFMGPPGPQGQPGLP

C4P7Ca base sequence (SEQ ID NO: 9):

GGATTTCCCGGGGTTCCCGGGTGCCAAAGGGGATAAAGGTTCAAAGGGCGAAGTGGGTTT
CCCGGGTTTGGCTGGTAGCCCGGGTATCCCGGGTAGCAAAGGCGAACAGGGCTTTATGGGTCC
GCCAGGACCGCAGGGTCAACCGGGACTGCCGGGTTTTCCGGGCTTCCCGGGTGCGAAAGGCGA
TAAAGGTTCCAAGGGTGAAGTTGGTTTTCCGGGTCTTGCAGGCAGCCCGGGTATTCCGGGTTCC
AAGGGTGAACAGGGTTTCATGGGTCCACCGGGCCCACAAGGTCAGCCGGGTCTGCCTGGTTTC
CCGGGCTTCCCGGGTGCCAAAGGCGACAAAGGTAGCAAGGGCGAAGTTGGCTTTCCGGGTCTG
GCGGGTTCGCCGGGCATTCCGGGCTCGAAGGGCGAGCAGGGTTTCATGGGCCCACCGGGTCCG
CAGGGTCAGCCTGGCCTGCCGGGATTCCCAGGTTTTCCGGGAGCGAAAGGCGACAAGGGTAGT
AAGGGTGAGGTCGGTTTTCCAGGCTTGGCGGGCTCTCCCGGTATCCCGGGCTCTAAGGGCGAGC
AAGGCTTTATGGGTCCACCGGGTCCGCAAGGTCAACCTGGATTACCGGGATTCCCAGGCTTTCC
GGGCGCGAAAGGCGATAAAGGCAGCAAGGGTGAGGTGGGCTTCCCGGGCCTCGCGGGTAGCC
CGGGCATCCCGGGTAGCAAGGGTGAGCAGGGCTTCATGGGTCCTCCGGGTCCGCAGGGCCAAC
CGGGCCTGCCGGGATTCCCGGGTTTCCCGGGCGCTAAAGGCGACAAAGGCAGCAAGGGTGAGG
TTGGTTTTCCGGGTCTGGCAGGTAGCCCGGGCATTCCGGGCTCCAAGGGCGAACAGGGTTTTAT
GGGTCCACCGGGCCCTCAAGGTCAGCCGGGCCTGCCG

4) C4P7Cb amino acid sequence (the amino acid sequence of the repeating unit is Gfpgfpgakgdkgskgevgfpglagspgipgskgeqgfmgppgpq, SEQ ID NO: 10, and the number of repeating units is 8; the amino acid sequence of C4P7Cb is SEQ ID NO: 11):

Gfpgfpgakgdkgskgevgfpglagspgipgskgeqgfmgppgpq
Gfpgfpgakgdkgskgevgfpglagspgipgskgeqgfmgppgpq
Gfpgfpgakgdkgskgevgfpglagspgipgskgeqgfmgppgpq
Gfpgfpgakgdkgskgevgfpglagspgipgskgeqgfmgppgpq

Gfpgfpgakgdkgskgevgfpglagspgipgskgeqgfmgppgpq
Gfpgfpgakgdkgskgevgfpglagspgipgskgeqgfmgppgpq
Gfpgfpgakgdkgskgevgfpglagspgipgskgeqgfmgppgpq
Gfpgfpgakgdkgskgevgfpglagspgipgskgeqgfmgppgpq

C4P7Cb base sequence (SEQ ID NO: 12):

GGATTTCCCGGGTTTCCGGGTGCGAAAGGCGACAAGGGTTCCAAGGGTGAAGTTGGTTTT
CCGGGGCTCGCTGGTAGCCCGGGCATCCCGGGTAGCAAGGGCGAACAGGGTTTCATGGGCCCA
CCGGGCCCGCAGGGTTTTCCGGGTTTCCCGGGCGCGAAAGGCGACAAAGGCAGCAAGGGTGAA
GTGGGCTTTCCGGGCCTGGCGGGTAGTCCGGGCATCCCGGGCTCGAAGGGCGAACAGGGCTTT
ATGGGCCCACCAGGCCCACAGGGCTTCCCGGGATTCCCGGGTGCCAAAGGCGATAAAGGTTCA
AAGGGCGAAGTCGGTTTCCCGGGTCTGGCAGGCTCTCCGGGCATCCCGGGCTCTAAGGGTGAA
CAAGGTTTCATGGGTCCACCGGGCCCGCAGGGCTTTCCGGGTTTTCCGGGTGCGAAAGGCGAC
AAGGGCAGCAAAGGTGAGGTTGGCTTTCCGGGCTTGGCGGGTAGCCCGGGTATCCCGGGTAGC
AAGGGTGAGCAGGGTTTCATGGGTCCGCCTGGACCGCAAGGTTTCCCGGGTTTCCCGGGTGCG
AAAGGTGATAAAGGTAGCAAGGGCGAGGTAGGTTTTCCGGGTCTGGCAGGCTCCCCGGGCATT
CCGGGCTCTAAGGGTGAGCAGGGTTTCATGGGTCCGCCAGGTCCGCAAGGTTTCCCGGGATTTC
CGGGTGCTAAAGGTGATAAAGGTTCCAAAGGTGAAGTGGGTTTTCCAGGCCTGGCCGGCAGCC
CGGGCATTCCGGGCTCCAAGGGCGAGCAGGGCTTCATGGGTCCGCCTGGCCCTCAAGGCTTCCC
CGGCTTCCCGGGAGCTAAGGGCGACAAAGGTTCCAAGGGCGAGGTGGGCTTCCCGGGCTTGGC
GGGTAGCCCGGGCATTCCGGGTAGCAAGGGTGAGCAAGGCTTTATGGGTCCGCCAGGTCCGCA
AGGGTTCCCGGGATTCCCGGGTGCGAAAGGTGATAAAGGCTCGAAGGGTGAGGTTGGTTTTCC
GGGTCTGGCAGGTAGCCCGGGGATTCCGGGTAGCAAAGGCGAACAAGGTTTCATGGGTCCTCC
GGGTCCACAG

5) C4P7Cc amino acid sequence (the amino acid sequence of the repeating unit is Gfpgfpgakgdkgskgevgfpglagspgipgskgeqgfm, SEQ ID NO: 13, and the number of repeating units is 8; the amino acid sequence of C4P7Cc is SEQ ID NO: 14):

|Gfpgfpgakgdkgskgevgfpglagspgipgskgeqgfm
Gfpgfpgakgdkgskgevgfpglagspgipgskgeqgfm
Gfpgfpgakgdkgskgevgfpglagspgipgskgeqgfm
Gfpgfpgakgdkgskgevgfpglagspgipgskgeqgfm
Gfpgfpgakgdkgskgevgfpglagspgipgskgeqgfm
Gfpgfpgakgdkgskgevgfpglagspgipgskgeqgfm
Gfpgfpgakgdkgskgevgfpglagspgipgskgeqgfm
Gfpgfpgakgdkgskgevgfpglagspgipgskgeqgfm

C4P7Cc base sequence (SEQ ID NO: 15):

GGATTTCCCGGGTTCCCGGGTGCGAAAGGTGATAAAGGCAGCAAGGGTGAAGTCGGTTT
TCCGGGTCTGGCAGGCAGCCCGGGTATCCCGGGTAGCAAAGGCGAACAGGGCTTTATGGGTTT

CCCGGGCTTCCCAGGTGCGAAGGGCGATAAAGGTTCGAAAGGTGAGGTAGGTTTCCCGGGTTT
AGCAGGTTCCCCGGGCATTCCGGGCAGCAAGGGTGAACAGGGTTTCATGGGCTTTCCGGGCTTC
CCAGGAGCTAAAGGCGACAAAGGTTCTAAGGGTGAAGTGGGCTTCCCGGGTCTGGCTGGTAGC
CCGGGCATCCCGGGCTCCAAGGGTGAGCAGGGTTTCATGGGTTTTCCGGGCTTCCCAGGCGCGA
AAGGCGACAAAGGCAGCAAGGGCGAGGTGGGTTTTCCGGGTTTGGCGGGTAGCCCGGGTATTC
CGGGTTCGAAGGGTGAACAAGGTTTCATGGGTTTTCCGGGATTCCCAGGCGCGAAAGGCGATA
AGGGCAGCAAGGGCGAGGTTGGCTTCCCGGGACTGGCCGGAAGCCCGGGTATCCCGGGATCTA
AGGGCGAACAAGGCTTTATGGGTTTCCCGGGTTTTCCTGGTGCGAAAGGCGATAAAGGCTCCA
AGGGCGAGGTTGGTTTTCCAGGCCTGGCTGGCTCTCCGGGCATTCCGGGTAGTAAGGGTGAGC
AGGGTTTTATGGGTTTTCCGGGCTTCCCGGGTGCAAAGGGTGACAAAGGTAGCAAGGGTGAAG
TTGGCTTTCCGGGTCTGGCGGGTTCCCCGGGCATTCCGGGTAGCAAAGGTGAGCAAGGTTTTAT
GGGTTTTCCGGGCTTCCCGGGTGCCAAAGGCGACAAAGGTAGCAAGGGAGAGGTGGGCTTCCC
GGGATTGGCGGGTTCCCCGGGCATCCCGGGCTCAAAGGGTGAACAGGGTTTCATG

6) C4P7Cd amino acid sequence (the amino acid sequence of the repeating unit is Gfpgfpgakgdkgskgevgfpglagspgipgskgeq, SEQ ID NO: 16, and the number of repeating units is 10; the amino acid sequence of C4P7Cd is SEQ ID NO: 17):

|Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq
Gfpgfpgakgdkgskgevgfpglagspgipgskgeq

C4P7Cd base sequence (SEQ ID NO: 18):

GGATTTCCCGGGTTCCCAGGTGCCAAGGGTGATAAAGGCAGCAAGGGCGAGGTGGGCTT
CCCGGGCCTGGCTGGTTCACCGGGCATTCCGGGCAGTAAGGGTGAACAGGGTTTTCCGGGTTTC
CCCGGCGCTAAAGGCGACAAGGGTAGCAAGGGGGAAGTAGGCTTCCCGGGTTTAGCTGGCTCT
CCGGGCATTCCGGGCTCCAAGGGCGAACAGGGTTTCCCGGGCTTTCCGGGAGCGAAAGGCGAC
AAAGGCAGCAAGGGCGAAGTTGGTTTCCCGGGTCTGGCAGGTTCTCCGGGTATCCCGGGTAGC
AAAGGCGAGCAGGGTTTCCCCGGCTTCCCGGGTGCGAAAGGTGATAAAGGTAGCAAGGGTGAG
GTGGGTTTCCCTGGCCTGGCCGGTTCTCCGGGCATCCCGGGTTCTAAGGGAGAACAAGGTTTTC
CGGGTTTTCCGGGTGCGAAAGGCGACAAAGGGTCCAAGGGTGAGGTTGGTTTTCCGGGTCTGG
CGGGTTCACCGGGCATCCCGGGTAGCAAGGGCGAACAAGGCTTTCCAGGCTTCCCGGGTGCGA
AAGGTGACAAAGGTTCCAAGGGCGAAGTTGGTTTTCCGGGTTTGGCGGGTAGCCCGGGTATCC
CGGGTTCCAAGGGTGAGCAAGGTTTTCCGGGATTCCCGGGTGCCAAAGGTGACAAAGGTTCGA

AGGGTGAAGTGGGTTTTCCAGGCTTGGCGGGTAGCCCGGGTATCCCGGGTTCCAAGGGTGAGC

AGGGCTTCCCGGGTTTCCCGGGTGCAAAAGGTGATAAAGGCTCCAAAGGTGAGGTGGGCTTCC

CTGGTCTCGCGGGTAGCCCGGGCATCCCGGGTAGCAAAGGTGAGCAGGGTTTCCCGGGCTTTCC

AGGAGCTAAAGGGGATAAAGGCAGCAAGGGCGAGGTTGGCTTCCCGGGTCTGGCGGGTTCGCC

GGGCATTCCGGGCAGCAAAGGCGAACAGGGCTTTCCGGGTTTTCCGGGTGCAAAGGGTGATAA

AGGTAGCAAGGGCGAGGTCGGTTTTCCGGGCCTGGCAGGTAGCCCAGGCATTCCGGGTTCGAA

GGGCGAACAA

7) C4P7Ce amino acid sequence (the amino acid sequence of the repeating unit is Gakgdkgskgevgfpglagspgipgskgeqgfmgppgpqgqpglp, SEQ ID NO: 19, and the number of repeating units is 8; the amino acid sequence of C4P7Ce is SEQ ID NO: 20):

|Gakgdkgskgevgfpglagspgipgskgeqgfmgppgpqgqpglp

Gakgdkgskgevgfpglagspgipgskgeqgfmgppgpqgqpglp

Gakgdkgskgevgfpglagspgipgskgeqgfmgppgpqgqpglp

Gakgdkgskgevgfpglagspgipgskgeqgfmgppgpqgqpglp

Gakgdkgskgevgfpglagspgipgskgeqgfmgppgpqgqpglp

Gakgdkgskgevgfpglagspgipgskgeqgfmgppgpqgqpglp

Gakgdkgskgevgfpglagspgipgskgeqgfmgppgpqgqpglp

Gakgdkgskgevgfpglagspgipgskgeqgfmgppgpqgqpglp

C4P7Ce base sequence (SEQ ID NO: 21):

GGGGCTAAAGGAGACAAGGGCAGCAAGGGCGAGGTTGGTTTCCCGGGCTTGGCGGGTTC

TCCGGGTATCCCGGGCTCCAAGGGTGAACAGGGTTTTATGGGTCCGCCAGGTCCGCAGGGCCA

ACCTGGTCTGCCGGGCGCGAAAGGTGACAAAGGTAGCAAGGGCGAAGTTGGTTTTCCGGGCCT

GGCCGGCAGCCCGGGCATTCCGGGCTCCAAAGGCGAGCAAGGCTTCATGGGTCCGCCTGGCCC

ACAGGGTCAACCGGGTCTGCCGGGCGCAAAAGGCGATAAAGGCAGCAAGGGCGAAGTAGGTT

TCCCGGGCCTTGCTGGCTCTCCGGGTATTCCGGGTAGCAAGGGTGAACAGGGTTTTATGGGCCC

ACCGGGACCACAGGGTCAACCGGGTCTGCCGGGTGCTAAAGGTGATAAAGGCAGCAAGGGCG

AGGTTGGTTTTCCGGGATTGGCGGGTTCTCCGGGCATCCCGGGTTCCAAGGGCGAACAAGGTTT

CATGGGACCGCCTGGCCCACAGGGTCAACCGGGTCTGCCTGGTGCAAAAGGCGACAAAGGTAG

CAAAGGCGAGGTCGGTTTTCCGGGCCTCGCGGGTAGCCCGGGAATCCCGGGTAGTAAGGGCGA

ACAGGGTTTCATGGGTCCACCGGGCCCACAGGGTCAGCCGGGTTTACCGGGTGCCAAAGGCGA

TAAAGGGTCGAAGGGCGAGGTGGGCTTCCCGGGTCTGGCAGGCAGCCCGGGTATTCCGGGCTC

CAAGGGTGAGCAGGGTTTTATGGGTCCACCGGGACCGCAAGGTCAACCGGGTCTGCCGGGCGC

TAAAGGCGATAAGGGTAGCAAAGGCGAGGTGGGCTTCCCGGGTCTGGCGGGTAGCCCGGGCAT

TCCGGGTTCCAAGGGTGAGCAGGGTTTCATGGGTCCGCCTGGTCCTCAAGGCCAGCCGGGGCT

GCCCGGCGCGAAAGGTGACAAAGGTAGCAAGGGCGAAGTGGGTTTTCCGGGTTTGGCGGGTTC

ACCGGGCATCCCGGGCTCGAAGGGTGAACAGGGTTTCATGGGTCCTCCGGGCCCGCAGGGACA

ACCGGGACTGCCG

8) C4P7Cg amino acid sequence (the amino acid sequence of the repeating unit is Gakgdkgskgevgfpglagspgipgskgeqgfm, SEQ ID NO: 22, and the number of repeating units is 10; the amino acid sequence of C4P7Cg is SEQ ID NO: 23):

|Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

Gakgdkgskgevgfpglagspgipgskgeqgfm

C4P7Cg base sequence (SEQ ID NO: 24):

GGGGCTAAAGGAGACAAAGGTTCGAAAGGCGAGGTGGGCTTCCCAGGTCTGGCCGGTTC
CCCGGGCATTCCGGGTAGCAAAGGCGAACAAGGTTTCATGGGTGCTAAAGGCGATAAAGGTAG
CAAGGGTGAGGTTGGCTTCCCAGGCCTGGCTGGTTCGCCGGGCATTCCGGGCTCTAAGGGTGA
ACAAGGTTTCATGGGTGCAAAAGGTGATAAGGGTAGCAAGGGAGAAGTCGGTTTTCCGGGATT
GGCGGGTAGCCCGGGTATCCCGGGCAGCAAGGGCGAGCAGGGTTTTATGGGTGCAAAGGGCG
ACAAAGGTAGCAAGGGTGAGGTGGGCTTTCCGGGCCTCGCGGGTAGCCCTGGCATCCCGGGTT
CCAAAGGTGAGCAAGGCTTCATGGGTGCTAAAGGTGATAAAGGCTCCAAAGGTGAAGTGGGTT
TTCCGGGCCTGGCGGGTAGCCCGGGCATTCCGGGAAGCAAGGGCGAACAGGGTTTTATGGGCG
CGAAGGGTGATAAAGGTAGTAAGGGCGAAGTTGGTTTCCCGGGCCTGGCTGGCTCTCCGGGTA
TCCCGGGCTCCAAAGGCGAGCAGGGTTTCATGGGTGCGAAAGGTGACAAGGGTAGCAAGGGTG
AGGTGGGTTTCCCAGGTTTGGCGGGTAGCCCGGGCATTCCGGGTAGCAAGGGTGAACAAGGTT
TCATGGGTGCGAAAGGTGACAAAGGCAGCAAGGGCGAGGTTGGTTTCCCGGGTCTGGCGGGTA
GCCCGGGCATCCCGGGCTCTAAGGGCGAGCAGGGTTTTATGGGTGCCAAAGGCGACAAGGGCT
CAAAGGGTGAAGTCGGTTTTCCGGGTTTAGCCGGTTCCCCGGGCATCCCGGGTTCTAAGGGTGA
ACAGGGCTTCATGGGCGCGAAAGGAGATAAAGGCAGCAAAGGGGAAGTTGGTTTTCCAGGCCT
GGCAGGCTCGCCGGGTATCCCGGGTTCCAAGGGCGAGCAGGGTTTTATG

9) C4P7Ea amino acid sequence (the amino acid sequence of the repeating unit is Glpgtpgptgpagqkgepgsdgipgsagekgepglp, SEQ ID NO: 25, and the number of repeating units is 10; the amino acid sequence of C4P7Ea is SEQ ID NO: 26):

|Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

Glpgtpgptgpagqkgepgsdgipgsagekgepglp

C4P7Ea base sequence (SEQ ID NO: 27):

GGACTACCCGGGACTCCGGGCCCAACCGGCCCAGCAGGCCAAAAGGGTGAACCGGGGTC
CGACGGCATTCCGGGCAGCGCAGGTGAGAAAGGCGAACCGGGCTTGCCGGGGTTGCCGGGAA
CCCCGGGTCCGACCGGTCCAGCTGGCCAGAAAGGTGAGCCAGGAAGCGATGGCATCCCGGGCT
CGGCCGGTGAAAAAGGCGAGCCGGGTCTCCCGGGTCTGCCGGGAACCCCGGGTCCGACAGGCC
CGGCTGGTCAGAAGGGCGAGCCGGGTAGCGACGGCATCCCGGGCAGCGCTGGTGAAAAAGGT
GAGCCCGGTCTGCCGGGCTTGCCCGGCACCCCGGGACCGACGGGCCCAGCAGGCCAGAAGGGC
GAACCGGGTTCGGATGGTATTCCGGGCTCTGCCGGTGAGAAAGGCGAGCCCGGCTTGCCAGGC
CTGCCTGGCACCCCGGGTCCGACCGGTCCGGCGGGCCAAAAAGGCGAGCCGGGTAGCGATGGT
ATCCCGGGCTCAGCCGGTGAGAAGGGTGAGCCGGGCCTGCCGGGCCTGCCTGGTACGCCGGGT
CCGACCGGTCCGGCGGGCCAAAAGGGTGAACCGGGCTCCGACGGCATTCCGGGTTCTGCGGGT
GAGAAAGGTGAACCGGGCCTGCCGGGCCTGCCCGGCACCCCGGGTCCTACGGGTCCGGCTGGT
CAGAAAGGCGAGCCGGGCTCCGATGGCATTCCGGGTTCTGCGGGTGAGAAGGGTGAACCGGGC
TTGCCAGGTCTGCCGGGCACCCCGGGTCCGACGGGTCCGGCGGGTCAGAAGGGTGAGCCGGGT
TCCGATGGTATCCCGGGCAGCGCGGGAGAAAAAGGTGAACCGGGTCTGCCGGGTCTTCCGGGT
ACTCCGGGTCCGACCGGCCCTGCGGGTCAGAAGGGTGAGCCGGGAAGCGACGGCATCCCGGGC
AGCGCGGGGGAGAAGGGTGAACCGGGTTTACCTGGCCTGCCGGGAACCCCGGGCCCTACCGGT
CCGGCGGGTCAAAAGGGCGAACCGGGCAGCGACGGTATCCCGGGTAGCGCAGGCGAAAAAGG
TGAACCGGGCCTGCCG

10) C4P7Eb amino acid sequence (the amino acid sequence of the repeating unit is Gptgpagqkgepgsdgipgsagekgepglp, SEQ ID NO: 28, and the number of repeating units is 10; the amino acid sequence of C4P7Eb is SEQ ID NO: 29):

|Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

Gptgpagqkgepgsdgipgsagekgepglp

C4P7Eb base sequence (SEQ ID NO: 30):

GGTCCCACAGGACCGGCAGGCCAGAAAGGTGAGCCGGGTTCCGACGGCATCCCGGGTTC GGCGGGTGAGAAAGGCGAGCCGGGTTTACCGGGTCCGACCGGTCCCGCGGGTCAAAAGGGCG AGCCGGGTAGCGATGGCATTCCGGGTTCTGCGGGTGAAAAGGGCGAACCGGGCCTCCCGGGTC CTACCGGTCCGGCGGGTCAGAAAGGCGAACCGGGCAGCGATGGCATCCCGGGCAGCGCGGGC GAGAAAGGCGAACCGGGCCTGCCGGGCCCGACCGGACCAGCTGGGCAAAAAGGTGAACCGGG CAGCGACGGCATCCCGGGTTCTGCAGGCGAGAAAGGTGAACCAGGCCTGCCGGGACCGACCGG TCCGGCAGGCCAGAAAGGTGAGCCTGGCAGTGATGGTATTCCGGGTTCTGCCGGTGAAAAAGG TGAGCCGGGCCTGCCGGGGCCAACGGGCCCAGCCGGACAAAAAGGTGAGCCGGGTTCCGACG GCATCCCGGGCTCCGCCGGTGAAAAGGGTGAGCCGGGCCTGCCTGGCCCAACGGGTCCGGCTG GCCAAAAGGGCGAGCCGGGTAGCGACGGCATTCCGGGCAGCGCGGGTGAGAAGGGTGAGCCG GGATTGCCGGGTCCGACTGGTCCTGCGGGCCAGAAGGGTGAACCGGGTTCCGACGGCATCCCC GGCTCGGCGGGTGAAAAGGGCGAACCGGGTCTGCCTGGTCCGACCGGCCCAGCGGGTCAGAAG GGTGAACCGGGTAGCGATGGAATCCCGGGTAGCGCTGGTGAAAAGGGCGAGCCGGGCCTGCC GGGTCCGACCGGTCCGGCAGGCCAGAAGGGTGAACCGGGTAGCGATGGTATTCCGGGTAGCGC GGGCGAAAAAGGTGAGCCGGGCTTGCCG

11) C4P7Ec amino acid sequence (the amino acid sequence of the repeating unit is Gfpgfpgakgdkgskgevgfpglagspgipgsk, SEQ ID NO: 31, and the number of repeating units is 10; the amino acid sequence of C4P7Ec is SEQ ID NO: 32):

|Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk
Gfpgfpgakgdkgskgevgfpglagspgipgsk

C4P7Ec base sequence (SEQ ID NO: 33):

GGATTTCCCGGGTTCCCAGGCGCAAAAGGTGATAAAGGCAGCAAGGGCGAGGTTGGTTT TCCAGGTTTAGCTGGTAGCCCGGGTATCCCGGGTAGCAAGGGCTTCCCGGGTTTTCCGGGTGCT AAAGGCGACAAAGGCTCCAAGGGCGAAGTCGGTTTCCCGGGTTTGGCGGGTAGCCCGGGTATC CCGGGTAGTAAGGGCTTTCCGGGATTCCCAGGCGCGAAAGGTGACAAAGGTAGCAAGGGCGA AGTTGGCTTCCCGGGTTTGGCGGGTTCCCGGGTATCCCGGGGTCCAAGGGCTTCCCCGGATTC CCGGGCGCGAAAGGCGATAAAGGTAGCAAGGGTGAAGTGGGTTTTCCGGGTCTCGCTGGCAGC CCGGGTATTCCGGGCTCCAAGGGCTTTCCAGGCTTTCCGGGTGCGAAAGGCGATAAAGGTAGC AAGGGTGAGGTGGGTTTTCCGGGTCTGGCAGGTAGCCCTGGCATCCCGGGCTCGAAGGGGTTC

CCGGGCTTCCCGGGAGCCAAGGGTGATAAAGGTTCTAAGGGTGAGGTCGGTTTTCCGGGCCTG GCCGGTAGCCCTGGTATCCCGGGGAGCAAGGGTTTCCCGGGTTTTCCGGGTGCCAAAGGCGAT AAAGGCTCTAAGGGCGAGGTGGGCTTCCCCGGTCTGGCGGGTAGCCCGGGTATTCCGGGTTCT AAGGGCTTCCCGGGTTTTCCGGGTGCGAAAGGTGACAAGGGCTCCAAGGGTGAAGTTGGTTTT CCGGGTCTGGCTGGTAGCCCGGGTATCCCGGGTAGCAAGGGCTTCCCGGGTTTCCCGGGCGCG AAAGGCGACAAAGGTTCAAAGGGTGAAGTTGGTTTTCCTGGCCTGGCAGGCAGCCCGGGCATT CCGGGTTCCAAAGGTTTTCCGGGCTTCCCGGGTGCGAAAGGTGACAAAGGCTCGAAGGGTGAG GTGGGCTTCCCGGGTCTGGCAGGTTCTCCTGGCATTCCGGGTTCGAAA

Each of the aforementioned encoding nucleotide sequences was commercially synthesized. Subsequently, each of the above encoding nucleotide sequences (a collagen-processing enzyme cleavage site with the amino acid sequence of ENLYFQ and the nucleotide sequence of GAAAACCTGTATTTCCAG was added at the 5' end) was inserted between the KpnI and XhoI cleavage sites of the pET-28a-Trx-His expression vector to generate a recombinant expression plasmid.

3. The successfully constructed expression plasmid was transformed into *E. coli* competent cell BL21 (DE3). Specifically, the process was as follows: (1) taking out *E. coli* competent cells BL21 (DE3) from an ultra-low temperature refrigerator and placing them on ice. Once the cells were half-thawed, pipette 2 μl of the plasmid to be transformed into the E. coli competent cells BL21 (DE3) and gently mix 2-3 times. (2) Placing the mixture on ice in an ice bath for 30 minutes, then heat shocking in a water bath at 42°C for 45-90 s, and removing the mixture and placing it on ice in an ice bath for 2 minutes. (3) Transferring the mixture to a biosafety cabinet, adding 700 μl of liquid LB medium, and then incubating at 37°C and at 220 rpm for 60 min. (4) Taking 200 μl of the bacterial solution and evenly spreading it on a LB plate containing ampicillin sodium. (5) Incubating the plate in an incubator at 37°C for 15-17 h, until colonies of uniform size had grown.

4. 5-6 single colonies were picked from the LB plate with transformed cells and placed in shake flasks containing LB medium supplemented with antibiotic stock solution, followed by incubation in a shaker at 220 rpm and a constant temperature of 37°C for 7 hours. The cultured shake flask was then cooled to 16°C, and IPTG was added to induce expression for a specific period. The bacterial solution was dispensed into centrifuge bottles and centrifuged at 8000 rpm and 4°C for 10 minutes. The bacterial cells were collected, the bacterial cell weight was recorded, and samples (labeled "bacterial solution") were taken for electrophoresis detection.

5. The collected bacterial cells were resuspended in an equilibrium working solution (200 mM sodium chloride, 25 mM Tris, 20 mM imidazole at pH 8.0), and the bacterial solution was cooled to ≤ 15 °C. The solution was homogenized by two rounds of high-pressure homogenization (samples were taken after each round and labeled "homogenization 1" and "homogenization 2", respectively), and the bacterial solution was collected after homogenization. The homogenized bacterial solution was divided into centrifuge bottles and centrifuged at 17,000 rpm and 4°C for 30 minutes. The supernatant was collected, and both the supernatant (labeled "Supernatant") and the pellets were taken for electrophoresis detection.

6. Purifying and enzymatically digesting the recombinant type IV humanized collagen. Specifically, the processes were as follows. (1) Crude purification: a. Washing the column material (Ni6FF, Cytiva) with water for 5 CVs. b. Equilibrating the column material with equilibrium solution (200 mM sodium chloride, 25 mM Tris, 20 mM imidazole, at pH 8.0) for 5 CVs. c. Loading: loading the obtained after centrifugation onto the column material until the liquid flowed through the column material completely, and then taking the flow through (labeled "Flow through") for electrophoresis detection. d. Washing impurity proteins: Adding 25 mL of washing solution (200 mM sodium chloride, 25 mM Tris, 20 mM imidazole) until the liquid flowed through completely, and taking the impurity-washing flow through (labeled "Impurity-washing") for electrophoresis detection. e. Collecting the target protein: adding 20 mL of eluate (200mM sodium chloride, 25mM Tris, 250mM imidazole, at pH 8.0), and collecting the flow-through liquid (marked: elution), detecting the protein concentration and calculating the protein amount, and performing electrophoresis detection. f. Washing the column material with 1 M imidazole working solution (labeled "Washing with 1 M"). g. Washing the column material with purified water. (2) Enzymatically digesting: according to the ratio of total protein amount to total TEV enzyme amount of 50: 1, adding TEV enzyme for digestion at 16°C for 4 h, and sampling for electrophoresis detection (labeled "After digestion"). Putting the enzymatically digested protein solution into a dialysis bag and dialyzing at 4°C for 2 h, then transferring it to a fresh dialysate for dialysis at 4°C overnight (labeled "Liquid exchange").

(3) Fine purification: a. equilibrating the column material (Capto Q, Cytiva): equilibrating the column material using solution A (20 mM Tris, 20 mM sodium chloride, at pH 8.0) at a flow rate of 10 ml/min. b. Loading: loading the sample at a flow rate of 5 ml/min and collecting the flow through (labeled "QFL"), and performing electrophoresis detection. c. Gradient eluting: Setting 0-15% solution B (20 mM Tris, 1 M sodium chloride, at pH 8.0) for 2 min followed by holding for 3 CVs, 15-30% solution B for 2 min followed by holding for 3 CVs, 30-50% solution B for 2 min followed by holding 3 CVs, respectively, collecting the peak and performing electrophoresis detection (labeled "Washing with solution B"). d. Washing the column material. The protein was stored at 4°C.

7. Electrophoretic detection

Specifically, the processes were as follows. 40 $\mu$L of sample solution was taken, and 10 $\mu$L of 5$\times$ protein loading buffer (250 mM Tris-HCl at pH 6.8, 10% SDS, 0.5% bromophenol blue, 50% glycerol, 5% $\beta$-mercaptoethanol) was added. The mixture was heated in boiling water at 100°C for 10 minutes, then 10 $\mu$L was loaded into each well of an SDS-PAGE protein gel. The gel was run at 80 V for 2 hours, stained for 20 minutes using Coomassie brilliant blue staining solution (0.1% Coomassie brilliant blue R-250, 25% isopropanol, 10% glacial acetic acid), and then decolorized using protein decolorization solution (10% acetic acid, 5% ethanol).

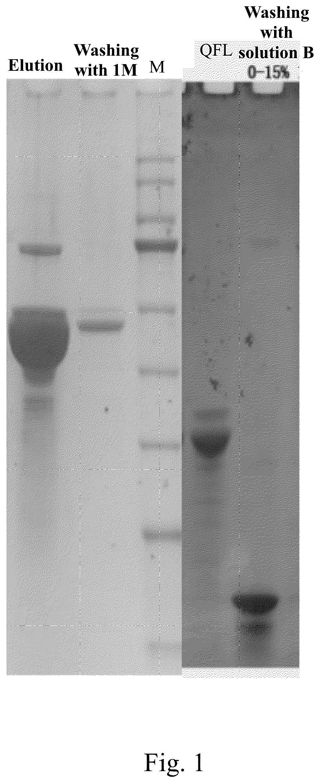

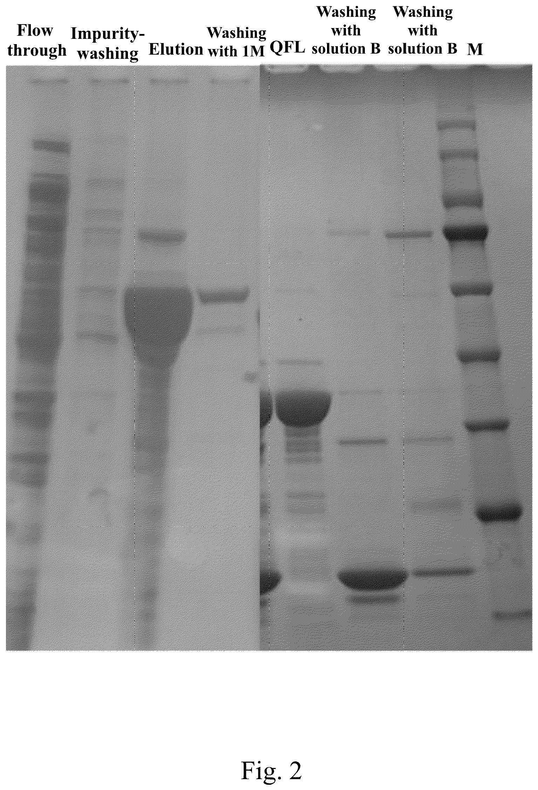

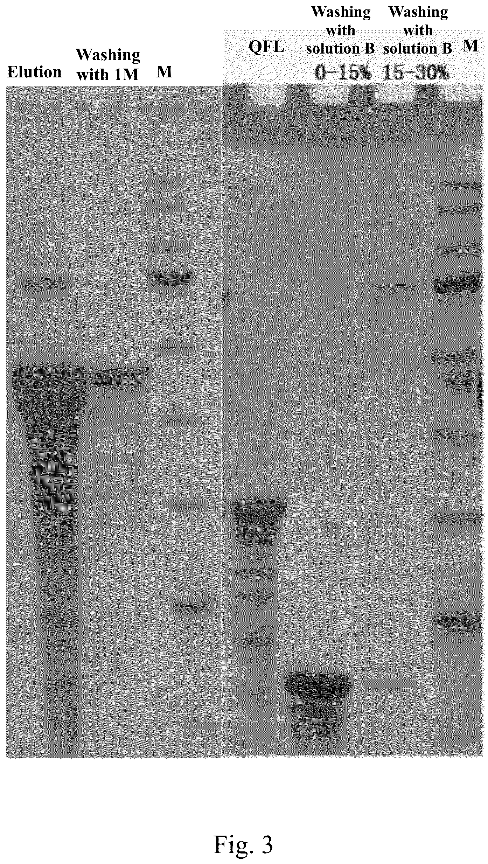

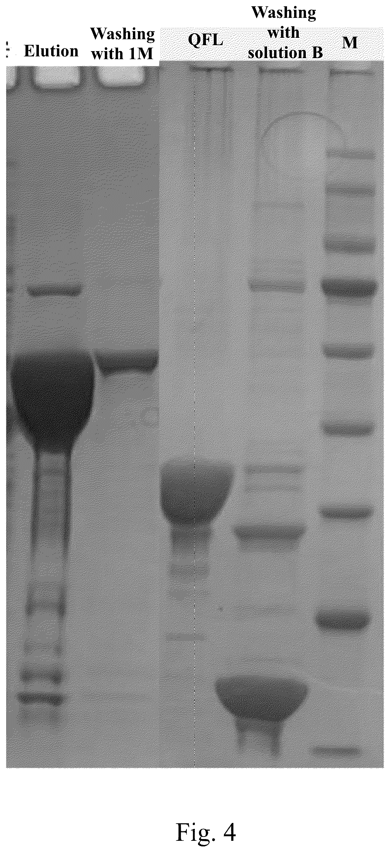

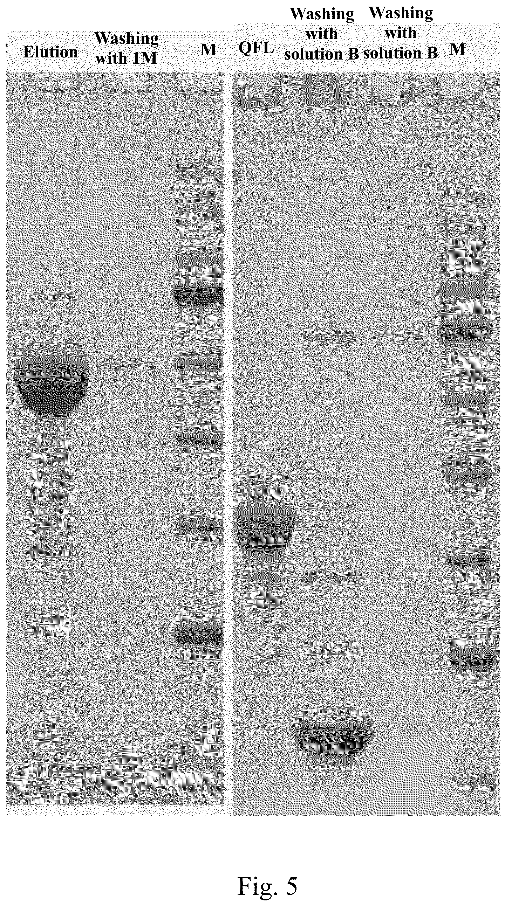

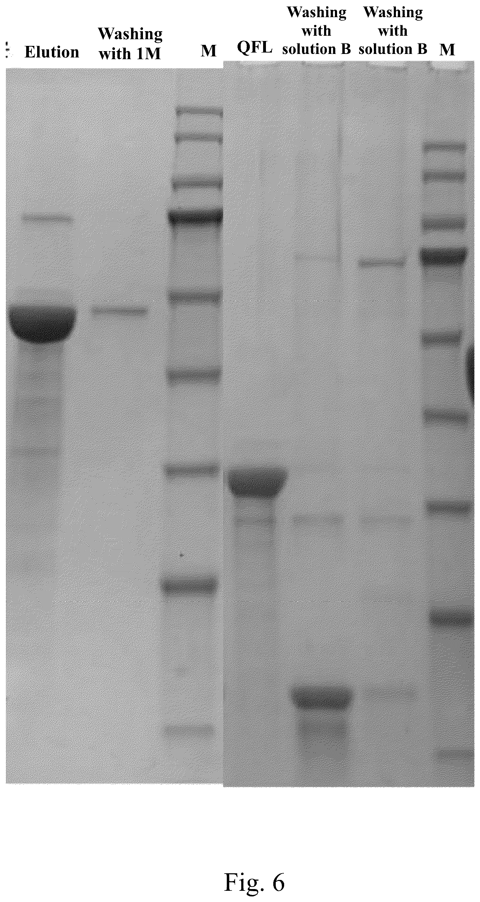

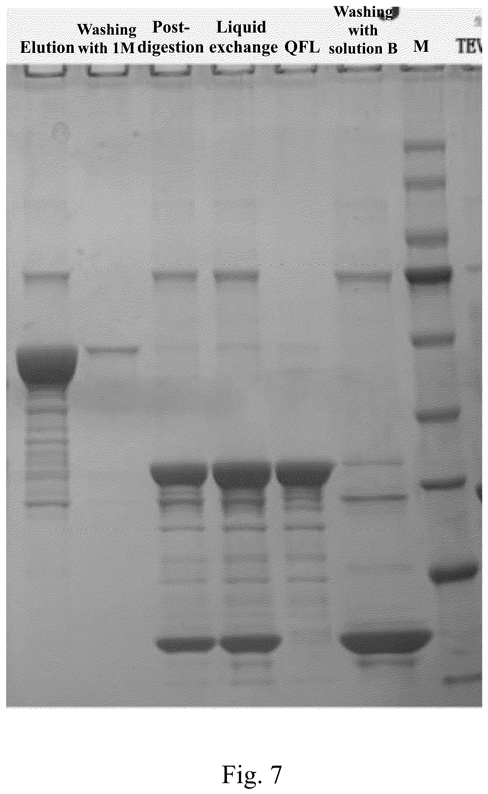

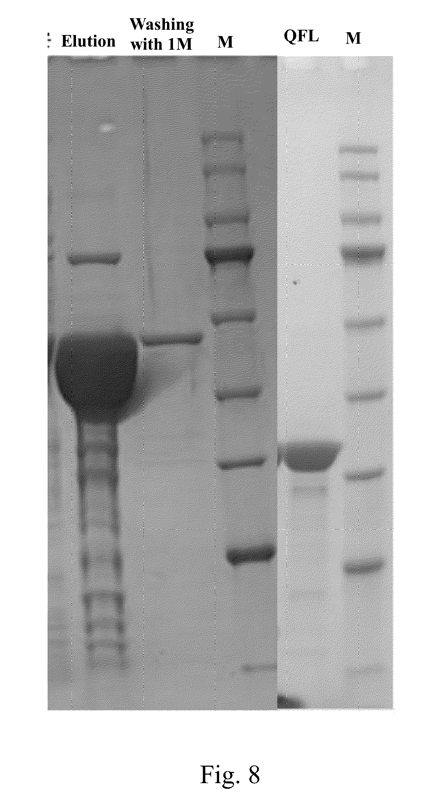

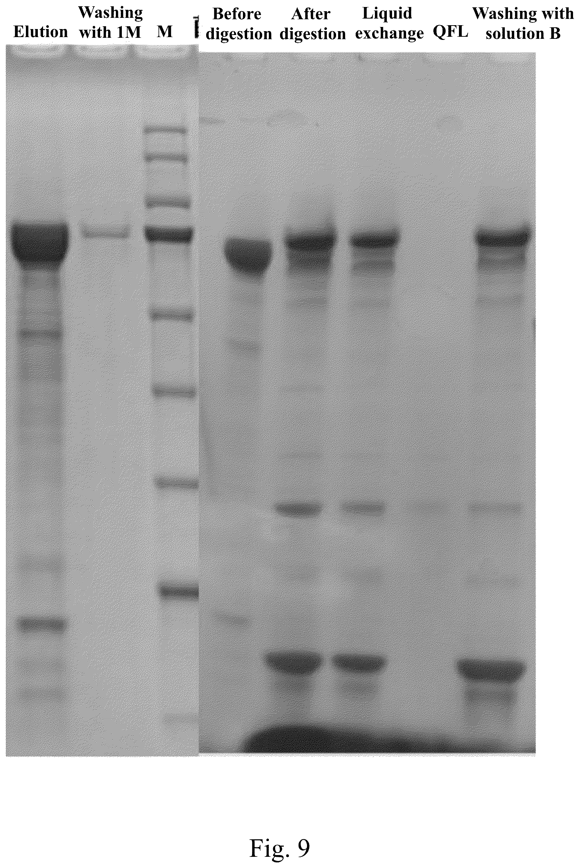

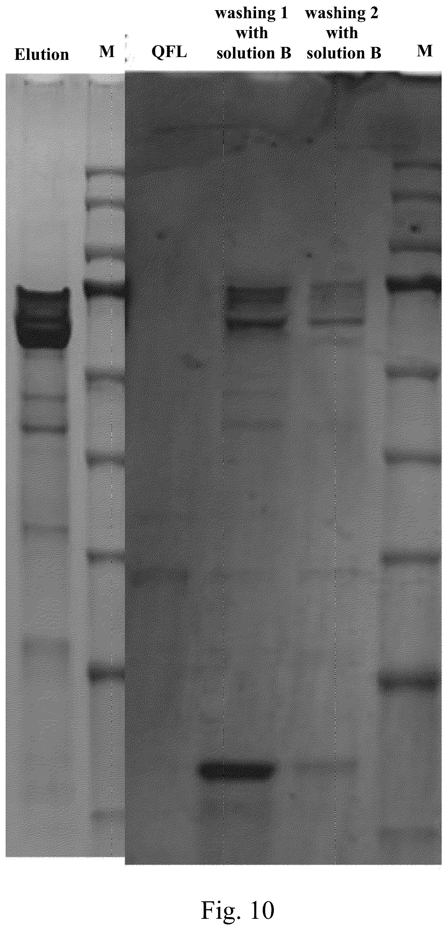

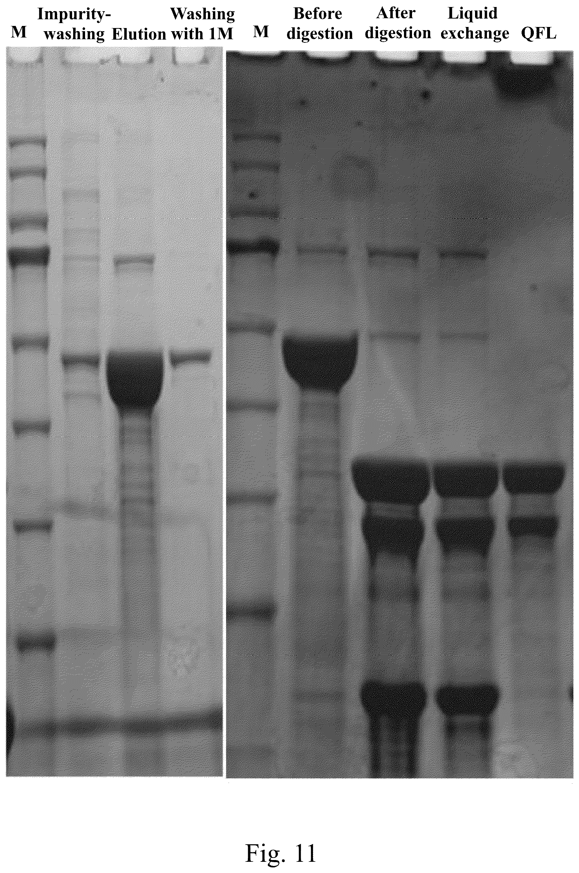

Fig. 1 shows the results of electrophoresis detection of C4P7Ca. Fig. 2 shows the results of electrophoresis detection of C4P7Cb. Fig. 3 shows the results of electrophoresis detection of C4P7Cc. Fig. 4 shows the results of electrophoresis detection of C4P7Cd. Fig. 5 shows the results of electrophoresis detection of C4P7Ce. Fig. 6 shows the results of electrophoresis detection of C4P7Cf. Fig. 7 shows the results of electrophoresis detection of C4P7Cg. Fig. 8 shows the results of electrophoresis detection of C4P7Ch. Fig. 9 shows the results of electrophoresis detection of C4P7Ea. Fig. 10 shows the results of electrophoresis detection of C4P7Eb. Fig. 11 shows the results of electrophoresis detection of C4P7Ec. Figs. 1-11 show that the actual molecular weights of the isolated proteins (C4P7Ca, C4P7Cb, C4P7Cc, C4P7Cd, C4P7Ce, C4P7Cf, C4P7Cg, C4P7Ch, C4P7Ea, C4P7Eb, and C4P7Ec) are consistent with their corresponding expected molecular weights, demonstrating that the proteins are correctly expressed.

**Example 2: Mass spectrometric detection of recombinant type IV humanized collagens**

Experimental method

| Instrument Name | Matrix-assisted laser desorption ionization-time of flight mass spectrometer MALDI-TOF/TOF UltraflextremeTM, Brucker, Germany | | |
|---|---|---|---|
| Matrix | CHCA | Laser energy | 125 |
| Data retrieval software | Mascot | Retrieval species | All species |
| Retrieval database | Provided sequences as library | | |

The protein samples (collagens C4P7Cf and C4P7Ch) were reduced with DTT and alkylated with iodoacetamide, followed by the addition of trypsin for enzymatic digestion overnight. The peptide fragments obtained after enzyme digestion were desalted using C18ZipTip, mixed with the matrix $\alpha$-cyano-4-hydroxycinnamic acid (CHCA), and spotted onto a plate. Finally, the matrix-assisted laser desorption ionization-time of flight mass spectrometer (MALDI-TOF/TOF UlraflextremeTM, Bruker, Germany) was used for analysis (see Protein J. 2016;35:212-7 for peptide fingerprinting technology).

Data retrieval was performed using the MS/MS Ion Search page from the local Mascot website. The protein identification results were obtained based on the primary mass spectrometry of the peptide fragments generated after enzymatic digestion. Detection parameters: trypsin enzymatic digestion, allowing two missed cleavage sites. The alkylation of cysteine was set as a fixed modification, and the oxidation of methionine was set as a variable modification.

The database used for identification was NCBprot.

Table 1: Molecular weights and corresponding polypeptides detected by mass spectrometry of recombinant type IV humanized collagen C4P7Cf

| Start-End | Observed value | Mr (expected value) | Peptide |
|---|---|---|---|
| 241-276 | 3353.6817 | 3352.6744 | GSKGEVGFPGLAGSPGIPGSKGEQGFMGPPGPQGAK |
| 244-276 | 3065.6004 | 3064.5932 | GEVGFPGLAGSPGIPGSKGEQGFMGPPGPQGAK |
| 244-279 | 3381.6741 | 3380.6668 | GEVGFPGLAGSPGIPGSKGEQGFMGPPGPQGARGDK |
| 262-276 | 1457.6553 | 1456.6480 | GEQGFMGPPGPQGAK |
| 262-276 | 1473.6731 | 1472.6658 | GEQGFMGPPGPQGAK |
| 262-279 | 1773.7801 | 1772.7728 | GEQGFMGPPGPQGAKGDK |
| 277-300 | 2199.0695 | 2198.0622 | GDKGSKGEVGFPGLAGSPGIPGSK |
| 280-300 | 1898.9500 | 1897.9427 | GSKGEVGFPGLAGSPGIPGSK |
| 280-312 | 3061.5039 | 3080.4966 | GSKGEVGFPGLAGSPGIPGSKGEQGFMGPPGPQ |
| 280-312 | 3097.4825 | 3096.4752 | GSKGEVGFPGLAGSPGIPGSKGEQGFMGPPGPQ |
| 283-300 | 1626.8268 | 1625.8196 | GEVGFPGLAGSPGIPGSK |
| 283-312 | 2825.2583 | 2824.2510 | GEVGFPGLAGSPGIPGSKGEQGFMGPPGPQ |
| 301-312 | 1217.5133 | 1216.5060 | GEQGFMGPPGPQ |

The coverage rate of the detected polypeptide segments was 99.04% compared with the theoretical sequence (GAKGDKGSKGEVGFPGLAGSPGIPGSKGEOGFMGPPGPOGAKGDKGSKGEVGFPGLAGSPGIPGS KGEQGFMG PPGPQGAKGDKGSKGEVGFPGLAGSPGIPGSKGEQGFMGPPGPQGAKGDKGSKGEVG FPGLAGSPGIPGSKGEQ GFMGPPGPQGAKGDKGSKGEVGFPGLAGSPGIPGSKGEQGFMGPPGPQG AKGDKGSKGEVGFPGLAGSPGIPGS KGEQGFMGPPGPQGAKGDKGSKGEVGFPGLAGSPGIPGSKG EOGFMGPPGPOGAKGDKGSKGEVGFPGLAGSP GIPGSKGEOGFMGPPGPQ, in which the covered part is underlined), thus the detection results were very credible.

Table 2: Molecular weights and corresponding polypeptides detected by mass spectrometry of recombinant type IV humanized collagen C4P7Ch

| Start-End | Observed value | Mr (expected value) | Peptide |
|---|---|---|---|
| 247-273 | 2469.2114 | 2468.2041 | GSKGEVGFPGLAGSPGIPGSKGEQGAK |
| 250-273 | 2197.0655 | 2196.0782 | GEVGFPGLAGSPGIPGSKGEQGAK |
| 250-276 | 2497.1996 | 24961923 | GEVGFPGLAGSPGIPGSKGEQGAKGDK |
| 277-297 | 1898.9493 | 1897.9421 | GSKGEVGFPGLAGSPGIPGSK |
| 280-297 | 1626.8349 | 1625.8276 | GEVGFPGLAGSPGIPGSK |
| 280-300 | 1940.9073 | 1939.9000 | GEVGFPGLAGSPGIPGSKGEQ |

The coverage rate of the detected polypeptide segments was 98% compared with the theoretical sequence (GAKGDKGSKGEVGFPGLAGSPGIPGSKGEQGAKGDKGSKGEVGFPGLAGSPGIPGSKGEQGAKGD KGSKGEVGF PGLAGSPGIPGSKGEQGAKGDKGSKGEVGFPGLAGSPGIPGSKGEQGAKGDKGSKGE VGFPGLAGSPGIPGSKGE QGAKGDKGSKGEVGFPGLAGSPGIPGSKGEQGAKGDKGSKGEVGFPGL AGSPGIPGSKGEQGAKGDKGSKGEVG FPGLAGSPGIPGSKGEQGAKGDKGSKGEVGFPGLAGSPGIP GSKGEQGAKGDKGSKGEVGFPGLAGSPGIPGSK-GEQ, in which the covered part is underlined), thus the detection results were very credible.

**Example 3: Detection of Bioactivity of Recombinant Type IV Humanized Collagen**

The methods for detecting collagen activity can be referred to in Juming Yao, Satoshi Yanagisawa, Tetsuo Asakura, Design, Expression and Characterization of Collagen-Like Proteins Based on the Cell Adhesive and Crosslinking Sequences Derived from Native Collagens, J Biochem. 136, 643-649(2004). The specific methods were performed as follows:

(1) The ultraviolet absorption method was used to detect the concentration of protein samples to be detected, including bovine type I collagen (National Institutes for Food and Drug Control, No. 380002), recombinant humanized collagens C4P7Cf and C4P7Ch provided by the present disclosure.

Specifically, the ultraviolet absorption of the samples at 215nm and 225nm was measured respectively, and the protein concentrations were calculated using the empirical formula C ($\mu$g/mL) = 144 $\times$ (A215-A225). Note that the detection should be performed when A215<1.5. The principle of this method is that the characteristic absorption of peptide bonds under far-ultraviolet light is detected, which is not affected by the chromophore content, with few interfering substances. The method is easy to operate, so it is suitable for the detection of human collagen and analogs thereof that do not develop color with Coomassie brilliant blue. (Refer to the reference Walker JM. The Protein Protocols Handbook, second edition. Humana Press. 43-45). After detecting the protein concentration, the concentration of all proteins to be tested was adjusted to 0.5 mg/mL with PBS.

(2) Sample preparation: the sample stock solution was used directly to conduct the experiment. The positive control bovine type I collagen (PC) was diluted to 1 mg/ml with D-PBS for future use; and the negative control was D-PBS buffer (NC).

(3) Coating: different concentrations of collagen (C4P7Cf or C4P7Ch) and the positive control and the negative control were added to an ELISA plate in a volume of 100$\mu$L per well, and 5 replicate wells were set in each group, then incubated at 4 °C overnight.

(4) Blocking: The supernatant was discarded, and 100 $\mu$L of 1% BSA (heat-inactivated at 56°C for 30 minutes) was added, followed by incubation at 37°C for 60 minutes. The supernatant was discarded, and the plate was washed 3 times with D-PBS solution.

(5) Cell seeding: $10^5$ well-cultured 3T3/NIH cells resuspended in D-PBS were added to each well and incubated at 37°C for 120 minutes. Each well was washed 3 times with D-PBS solution.

(6) Detection: the absorbance at OD450 nm was detected using CCK8 detection kit (manufacturer Beyotime Biotech Inc., product catalog number C0038). The adherence degree of the cells was calculated according to the following formula. The adherence rate of cells can reflect the cell-adhesion ability of collagen. The higher the cell adhesion ability, the better the external environment can be provided to the cells in a short time to facilitate cell adherence.

$$p = \frac{OD_1 - OD_0}{OD_2 - OD_0}$$

where:

P: relative cell adhesion ratio;
$OD_1$: the average ultraviolet absorbance at 450 nm of all replicate wells of the tested collagen sample;
$OD_2$: Average ultraviolet absorbance at 450 nm for all replicate wells of the control collagen sample;
$OD_0$: Average ultraviolet absorbance at 450 nm for all replicate wells of the blank control group.

(7) Statistical analysis: The statistical difference between the target recombinant humanized collagen and the negative control was statistically analyzed by two-tailed t-test, where *, $P < 0.05$; * *, $P < 0.01$; * * *, $P < 0.001$.

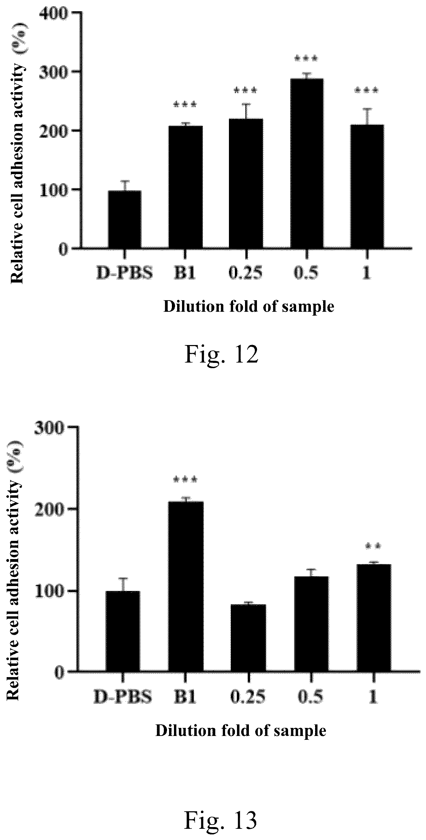

The results were shown in Figs. 11 and 12. As compared with the D-PBS group, the positive control had a significant effect in promoting cell adhesion, and the recombinant humanized collagen C4P7Cf and C4P7Ch also promote cell adhesion.

Although the present disclosure has been described with reference to illustrative embodiments, those skilled in the art will appreciate that various other changes, omissions and/or additions may be made and that elements of the described embodiments may be replaced with substantial equivalents without departing from the spirit and scope of the disclosure. In addition, many modifications can be made to adapt particular situations or materials to the teachings of the present disclosure without departing from the scope of the present disclosure. Accordingly, it is not intended herein to limit the disclosure to the specific embodiments disclosed for carrying out the disclosure, but rather it is intended that the disclosure will include all embodiments falling within the scope of the appended claims.

**Claims**

1. A recombinant collagen comprising one or more repeating units linked directly or via a linker, wherein the repeating units comprise an amino acid sequence selected from the group consisting of SEQ ID NO: 1 or 28, or a variant thereof, and wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence;

   preferably, wherein the repeating units are 2-50 repeating units, e.g., 2-45, 2-40, 2-35, 2-30, 2-25, 2-20, 2-15, 4-10 or 6-10 repeating units;
   preferably, wherein the linker comprises one or more amino acid residues, e.g., 1-10, 1-9, 1-8, 1-7, 1-6, 1-5, 1-4, 1-3 or 1-2 amino acid residues;
   preferably, wherein the mutation is selected from the group consisting of a substitution, an addition, an insertion or a deletion;
   preferably, wherein the substitution is conservative amino acid substitution;
   preferably, wherein the recombinant collagen is a recombinant humanized collagen; preferably a recombinant type IV collagen; preferably a recombinant human type IV collagen or a recombinant type IV humanized collagen;
   preferably, wherein the recombinant collagen has cell adhesion activity;
   preferably, wherein the variant of SEQ ID NO: 1 comprises the following mutations: the addition of GFPGFP (SEQ ID NO: 34) or the N-terminal truncated fragment of SEQ ID NO: 34 with a length of 1-5 amino acids at the N-terminus of the amino acid sequence of SEQ ID NO: 1, and/or addition of GFMGPPGPQGQPGLP (SEQ ID NO: 35) or the C-terminal truncated fragment of SEQ ID NO: 35 with a length of 1-14 amino acids at the C-terminus of the amino acid sequence of SEQ ID NO: 1, or the truncation of the amino acid sequence of SEQ ID NO: 1 at C-terminus by 1-5 amino acid residues;
   preferably, wherein the variant of SEQ ID NO: 28 comprises the following mutations: addition of Glpgtp (SEQ ID NO: 36) or the N-terminal truncated fragment thereof with a length of 1-5 amino acid residues at the N-terminus of the amino acid sequence of SEQ ID NO: 28;
   preferably, wherein the variant of SEQ ID NO: 1 is selected from the group consisting of SEQ ID NO: 4, 7, 10, 13, 16, 19, 22, or 31; and/or the variant of SEQ ID NO: 28 is SEQ ID NO: 25.

2. The recombinant collagen according to claim 1, comprising an amino acid sequence selected from the group consisting of SEQ ID NO: 2, 5, 8, 11, 14, 17, 20, 23, 26, 29 or 32, or a variant thereof, wherein the variant is (1) an amino acid sequence having one or more amino acid residue mutations in said amino acid sequence or (2) an amino acid sequence having at least 80%, 85%, 86%, 87%, 88%, 89%, 90%, 91%, 92%, 93%, 94%, 95%, 96%, 97%, 98%, 99% or 100% identity to said amino acid sequence;

   preferably, wherein the mutation is selected from the group consisting of a substitution, an addition, an insertion or a deletion;
   preferably, wherein the substitution is conservative amino acid substitution.

3. A nucleic acid encoding the recombinant collagen according to claim 1 or 2,

   preferably, wherein the nucleic acid comprises a codon-optimized nucleotide sequence,
   preferably, wherein the nucleotide sequence is a nucleotide sequence codon-optimized for expression in eukaryotic host cells or prokaryotic host cells, such as yeast or *E. coli;*
   preferably, wherein the nucleic acid comprises a nucleotide sequence selected from the group consisting of SEQ ID NO: 3, 6, 9, 12, 15, 18, 21, 24, 27, 30 or 33.

4. A vector comprising the nucleic acid according to claim 3,

   preferably, wherein the vector comprises an expression control element, nucleotides of a purification tag and/or nucleotides of a leader sequence operably linked to the nucleic acid,
   preferably, wherein the expression control element is selected from the group consisting of a promoter, a terminator or an enhancer;
   preferably, wherein the purification tag is selected from the group consisting of a His tag, a GST tag, an MBP tag, a SUMO tag or a NusA tag;
preferably, the vector is an expression vector or a cloning vector, preferably pET-28a(+).

5. A host cell comprising the nucleic acid according to claim 3 or the vector according to claim 4; preferably, wherein the host cell is a eukaryotic cell or a prokaryotic cell; preferably, wherein the eukaryotic cell is a yeast cell, an animal cell and/or an insect cell, and/or the prokaryotic cell is an *E. coli* cell, for example *E. coli* BL21.

6. A composition comprising one or more of the recombinant collagen according to claim 1 or 2, the nucleic acid according to claim 3, the vector according to claim 4 and the host cell according to claim 5, preferably the composition is a kit; preferably, the composition is one or more of a biological dressing, a human bionic material, a plastic surgery or beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive, preferably, wherein the composition is a composition for topical use, injection use, or oral use; preferably, the composition is a composition in the form of a solution, a lyophilized powder, a gel, a sponge, or a fiber.

7. Use of the recombinant collagen according to claim 1 or 2, the nucleic acid according to claim 3, the vector according to claim 4, the host cell according to claim 5, and/or the composition according to claim 6 in one or more of a biological dressing, a human bionic material, a plastic surgery or beauty material, an organoid culture material, a cardiovascular stent material, a coating material, a tissue injection filling material, an ophthalmic material, an obstetrics and gynecology biomaterial, a nerve repair and regeneration material, a liver tissue material and blood vessel repair and regeneration material, a 3D printed artificial organ biomaterial, a cosmetic raw material, a pharmaceutical excipient, and a food additive.

8. A method of facilitating cell adhesion comprising the step of contacting the recombinant collagen according to claim 1 or 2, the nucleic acid according to claim 3, the vector according to claim 4, the host cell according to claim 5 and/or the composition according to claim 6 with a cell, preferably the cell is an animal cell, preferably a mammalian cell, preferably a human cell.

9. A beauty method comprising administration of the recombinant collagen according to claim 1 or 2 to a subject, preferably, the administration is a topical administration, oral administration or injection administration; preferably, the subject is a human.

10. A method of producing the recombinant collagen according to claim 1 or 2, comprising:

(1) incubating the host cell according to claim 5 under a suitable culture condition;
(2) harvesting the host cell and/or culture medium comprising the recombinant collagen; and
(3) purifying the recombinant collagen;
preferably, wherein the host cell is an *E. coli* cell, preferably an *E. coli* BL21 (DE3) cell;
preferably, step (3) comprises purifying the recombinant collagen with a purification column, such as a nickel column, and/or cleaving the recombinant collagen with a collagen-processing enzyme.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

## INTERNATIONAL SEARCH REPORT

| International application No. |
|---|
| **PCT/CN2023/140632** |

**A. CLASSIFICATION OF SUBJECT MATTER**

C07K 14/78(2006.01)i; C12N15/09(2006.01)i; A61Q19/00(2006.01)i; A61K47/42(2017.01)i; A61L15/32(2006.01)i; A61L27/24(2006.01)i

According to International Patent Classification (IPC) or to both national classification and IPC

**B. FIELDS SEARCHED**

Minimum documentation searched (classification system followed by classification symbols)

IPC: C07K; C12N; A61K; A61Q; A61L

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science, GenBank, 中国专利生物序列检索系统, China Patents Biological Sequence Search System: 胶原, collagen, 4, IV, 本申请的SEQ ID NOs: 4, 7, 10, 13, 16, 19, 22, 31, 28和25

**C. DOCUMENTS CONSIDERED TO BE RELEVANT**

| Category* | Citation of document, with indication, where appropriate, of the relevant passages | Relevant to claim No. |
|---|---|---|
| X | CN 116478274 A (SHANXI JINBO BIO-PHARMACEUTICAL CO., LTD.) 25 July 2023 (2023-07-25) <br> entire document, in particular, claims, description, paragraph [0006], and abstract | 1-10 |
| Y | CN 116478274 A (SHANXI JINBO BIO-PHARMACEUTICAL CO., LTD.) 25 July 2023 (2023-07-25) <br> entire document, in particular, claims, description, paragraph [0006], and abstract | 1-10 |
| Y | NCBI. "PREDICTED: LOW QUALITY PROTEIN: collagen alpha-1(IV) chain [Rhinopithecus bieti]" <br> *NCBI Reference Sequence: XP_017723683.1, [retrieved from https://www.ncbi.nlm.nih.gov],* 24 August 2016 (2016-08-24), <br> entire document, and in particular, the sequences | 1-10 |

☐ Further documents are listed in the continuation of Box C.  ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **12 January 2024** | **25 January 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/140632** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

| International application No. |
| :--- |
| **PCT/CN2023/140632** |

| Patent document cited in search report | Publication date (day/month/year) | Patent family member(s) | Publication date (day/month/year) |
| :---: | :---: | :---: | :---: |
| CN 116478274 A | 25 July 2023 | None | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311391711
- WO 9425612 A
- WO 2010039889 A

### Non-patent literature cited in the description

- **NEEDLEMAN** ; **WUNSCH**. *J. Mol. Biol.*, 1970, vol. 48, 443-453
- **RICE et al.** *Trends Genet.*, 2000, vol. 16, 276-277
- **HUE et al.** *Journal of Bacteriology*, 1995, vol. 177, 3465-3471
- **GUO** ; **SHERMAN**. *Mol. Cellular Biol*, 1995, vol. 15, 5983-5990
- **SIMONEN** ; **PALVA**. *Microbiological Reviews*, 1993, vol. 57, 109-137
- **ROMANOS et al.** *Yeast*, vol. 8, 423-488
- **SAMBROOK et al.** Molecular Cloning: A Laboratory Manual. Cold Spring Harbor Laboratory Press, 1989
- *Protein J.*, 2016, vol. 35, 212-7
- **JUMING YAO** ; **SATOSHI YANAGISAWA** ; **TETSUO ASAKURA**. Design, Expression and Characterization of Collagen-Like Proteins Based on the Cell Adhesive and Crosslinking Sequences Derived from Native Collagens. *J Biochem.*, 2004, vol. 136, 643-649
- **WALKER JM.** The Protein Protocols Handbook. Humana Press, 43-45